(19) Europäisches Patentamt
European Patent Office
Office européen des brevets

(11) EP 2 459 712 B1

(12) **FASCICULE DE BREVET EUROPEEN**

(45) Date de publication et mention de la délivrance du brevet:
**17.06.2015 Bulletin 2015/25**

(21) Numéro de dépôt: **10747913.1**

(22) Date de dépôt: **20.07.2010**

(51) Int Cl.:
***C12N 9/04*** *(2006.01)* ***C12N 15/53*** *(2006.01)*
***C12N 15/52*** *(2006.01)*

(86) Numéro de dépôt international:
**PCT/FR2010/000522**

(87) Numéro de publication internationale:
**WO 2011/012779 (03.02.2011 Gazette 2011/05)**

(54) **MUTANTS DE LA PYRROLOQUINOLINE QUINONE GLUCOSE DÉSHYDROGÉNASE SOLUBLE**

MUTANTEN VON PYRROLOCHINOLINCHINON-ABHÄNGIGER LÖSLICHER GLUCOSE-DEHYDROGENASE

MUTANTS OF PYRROLOQUINOLINE QUININE-DEPENDENT SOLUBLE GLUCOSE DEHYDROGENASE

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **28.07.2009 FR 0903694**

(43) Date de publication de la demande:
**06.06.2012 Bulletin 2012/23**

(73) Titulaire: **Centre National de la Recherche Scientifique**
**75016 Paris (FR)**

(72) Inventeurs:
- **MANO, Nicolas**
  **F-33400 Talence (FR)**
- **STINES-CHAUMEIL, Claire**
  **F-33400 Talence (FR)**
- **DURAND, Fabien**
  **F-33000 Bordeaux (FR)**

(74) Mandataire: **Bernstein, Claire Jacqueline et al**
**Cabinet Orès**
**36, rue de Saint Pétersbourg**
**75008 Paris (FR)**

(56) Documents cités:
**EP-A1- 1 666 586** **WO-A1-02/34919**
**WO-A1-2006/085509**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 459 712 B1

Printed by Jouve, 75001 PARIS (FR)

SEQUENCE LISTING

<110> Centre National de la Recherche scientifique

<120> Mutants de la pyrroloquinoline quinone glucose déshydrogénase soluble

<130> F644 213WO

<160> 33

<170> PatentIn version 3.3

<210> 1
<211> 1389
<212> DNA
<213> Acinetobacter calcoaceticus

<220>
<221> CDS
<222> (1)..(1389)

<400> 1

gat gtt cct cta act cca tct caa ttt gct aaa gcg aaa tca gag aac 48
Asp Val Pro Leu Thr Pro Ser Gln Phe Ala Lys Ala Lys Ser Glu Asn
1 5 10 15

ttt gac aag aaa gtt att cta tct aat cta aat aag ccg cat gct ttg 96
Phe Asp Lys Lys Val Ile Leu Ser Asn Leu Asn Lys Pro His Ala Leu
20 25 30

tta tgg gga cca gat aat caa att tgg tta act gag cga gca aca ggt 144
Leu Trp Gly Pro Asp Asn Gln Ile Trp Leu Thr Glu Arg Ala Thr Gly
35 40 45

aag att cta aga gtt aat cca gag tcg ggt agt gta aaa aca gtt ttt 192
Lys Ile Leu Arg Val Asn Pro Glu Ser Gly Ser Val Lys Thr Val Phe
50 55 60

cag gta cca gag att gtc aat gat gct gat ggg cag aat ggt tta tta 240
Gln Val Pro Glu Ile Val Asn Asp Ala Asp Gly Gln Asn Gly Leu Leu
65 70 75 80

ggt ttt gcc ttc cat cct gat ttt aaa aat aat cct tat atc tat att 288
Gly Phe Ala Phe His Pro Asp Phe Lys Asn Asn Pro Tyr Ile Tyr Ile
85 90 95

tca ggt aca ttt aaa aat ccg aaa tct aca gat aaa gaa tta ccg aac 336
Ser Gly Thr Phe Lys Asn Pro Lys Ser Thr Asp Lys Glu Leu Pro Asn
100 105 110

caa acg att att cgt cgt tat acc tat aat aaa tca aca gat acg ctc 384
Gln Thr Ile Ile Arg Arg Tyr Thr Tyr Asn Lys Ser Thr Asp Thr Leu
115 120 125

gag aag cca gtc gat tta tta gca gga tta cct tca tca aaa gac cat 432
Glu Lys Pro Val Asp Leu Leu Ala Gly Leu Pro Ser Ser Lys Asp His
130 135 140

cag tca ggt cgt ctt gtc att ggg cca gat caa aag att tat tat acg 480
Gln Ser Gly Arg Leu Val Ile Gly Pro Asp Gln Lys Ile Tyr Tyr Thr
145 150 155 160

att ggt gac caa ggg cgt aac cag ctt gct tat ttg ttc ttg cca aat 528
Ile Gly Asp Gln Gly Arg Asn Gln Leu Ala Tyr Leu Phe Leu Pro Asn
165 170 175

caa gca caa cat acg cca act caa caa gaa ctg aat ggt aaa gac tat 576

```
Gln Ala Gln His Thr Pro Thr Gln Gln Glu Leu Asn Gly Lys Asp Tyr
            180                 185                 190

cac acc tat atg ggt aaa gta cta cgc tta aat ctt gat gga agt att        624
His Thr Tyr Met Gly Lys Val Leu Arg Leu Asn Leu Asp Gly Ser Ile
        195                 200                 205

cca aag gat aat cca agt ttt aac ggg gtg gtt agc cat att tat aca        672
Pro Lys Asp Asn Pro Ser Phe Asn Gly Val Val Ser His Ile Tyr Thr
    210                 215                 220

ctt gga cat cgt aat ccg cag ggc tta gca ttc act cca aat ggt aaa        720
Leu Gly His Arg Asn Pro Gln Gly Leu Ala Phe Thr Pro Asn Gly Lys
225                 230                 235                 240

tta ttg cag tct gaa caa ggc cca aac tct gac gat gaa att aac ctc        768
Leu Leu Gln Ser Glu Gln Gly Pro Asn Ser Asp Asp Glu Ile Asn Leu
                245                 250                 255

att gtc aaa ggt ggc aat tat ggt tgg ccg aat gta gca ggt tat aaa        816
Ile Val Lys Gly Gly Asn Tyr Gly Trp Pro Asn Val Ala Gly Tyr Lys
            260                 265                 270

gat gat agt ggc tat gct tat gca aat tat tca gca gca gcc aat aag        864
Asp Asp Ser Gly Tyr Ala Tyr Ala Asn Tyr Ser Ala Ala Ala Asn Lys
        275                 280                 285

tca att aag gat tta gct caa aat gga gta aaa gta gcc gca ggg gtc        912
Ser Ile Lys Asp Leu Ala Gln Asn Gly Val Lys Val Ala Ala Gly Val
    290                 295                 300

cct gtg acg aaa gaa tct gaa tgg act ggt aaa aac ttt gtc cca cca        960
Pro Val Thr Lys Glu Ser Glu Trp Thr Gly Lys Asn Phe Val Pro Pro
305                 310                 315                 320

tta aaa act tta tat acc gtt caa gat acc tac aac tat aac gat cca       1008
Leu Lys Thr Leu Tyr Thr Val Gln Asp Thr Tyr Asn Tyr Asn Asp Pro
                325                 330                 335

act tgt gga gag atg acc tac att tgc tgg cca aca gtt gca ccg tca       1056
Thr Cys Gly Glu Met Thr Tyr Ile Cys Trp Pro Thr Val Ala Pro Ser
            340                 345                 350

tct gcc tat gtc tat aag ggc ggt aaa aaa gca att act ggt tgg gaa       1104
Ser Ala Tyr Val Tyr Lys Gly Gly Lys Lys Ala Ile Thr Gly Trp Glu
        355                 360                 365

aat aca tta ttg gtt cca tct tta aaa cgt ggt gtc att ttc cgt att       1152
Asn Thr Leu Leu Val Pro Ser Leu Lys Arg Gly Val Ile Phe Arg Ile
    370                 375                 380

aag tta gat cca act tat agc act act tat gat gac gct gta ccg atg       1200
Lys Leu Asp Pro Thr Tyr Ser Thr Thr Tyr Asp Asp Ala Val Pro Met
385                 390                 395                 400

ttt aag agc aac aac cgt tat cgt gat gtg att gca agt cca gat ggg       1248
Phe Lys Ser Asn Asn Arg Tyr Arg Asp Val Ile Ala Ser Pro Asp Gly
                405                 410                 415

aat gtc tta tat gta tta act gat act gcc gga aat gtc caa aaa gat       1296
Asn Val Leu Tyr Val Leu Thr Asp Thr Ala Gly Asn Val Gln Lys Asp
            420                 425                 430

gat ggc tca gta aca aat aca tta gaa aac cca gga tct ctc att aag       1344
Asp Gly Ser Val Thr Asn Thr Leu Glu Asn Pro Gly Ser Leu Ile Lys
        435                 440                 445

ttc acc tat aag gct aag taa tac agt cgc att aaa aaa ccg atc           1389
```

Phe Thr Tyr Lys Ala Lys Tyr Ser Arg Ile Lys Lys Pro Ile
450 455 460

<210> 2
<211> 454
<212> PRT
<213> Acinetobacter calcoaceticus

<400> 2

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ser Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Asn Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Val Ser His Ile Tyr Thr
    210                 215                 220

Leu Gly His Arg Asn Pro Gln Gly Leu Ala Phe Thr Pro Asn Gly Lys
```

225 230 235 240

Leu Leu Gln Ser Glu Gln Gly Pro Asn Ser Asp Asp Glu Ile Asn Leu
245 250 255

Ile Val Lys Gly Gly Asn Tyr Gly Trp Pro Asn Val Ala Gly Tyr Lys
260 265 270

Asp Asp Ser Gly Tyr Ala Tyr Ala Asn Tyr Ser Ala Ala Ala Asn Lys
275 280 285

Ser Ile Lys Asp Leu Ala Gln Asn Gly Val Lys Val Ala Ala Gly Val
290 295 300

Pro Val Thr Lys Glu Ser Glu Trp Thr Gly Lys Asn Phe Val Pro Pro
305 310 315 320

Leu Lys Thr Leu Tyr Thr Val Gln Asp Thr Tyr Asn Tyr Asn Asp Pro
325 330 335

Thr Cys Gly Glu Met Thr Tyr Ile Cys Trp Pro Thr Val Ala Pro Ser
340 345 350

Ser Ala Tyr Val Tyr Lys Gly Gly Lys Lys Ala Ile Thr Gly Trp Glu
355 360 365

Asn Thr Leu Leu Val Pro Ser Leu Lys Arg Gly Val Ile Phe Arg Ile
370 375 380

Lys Leu Asp Pro Thr Tyr Ser Thr Thr Tyr Asp Asp Ala Val Pro Met
385 390 395 400

Phe Lys Ser Asn Asn Arg Tyr Arg Asp Val Ile Ala Ser Pro Asp Gly
405 410 415

Asn Val Leu Tyr Val Leu Thr Asp Thr Ala Gly Asn Val Gln Lys Asp
420 425 430

Asp Gly Ser Val Thr Asn Thr Leu Glu Asn Pro Gly Ser Leu Ile Lys
435 440 445

Phe Thr Tyr Lys Ala Lys
450

5

<210> 3
<211> 1389
<212> DNA
<213> Acinetobacter calcoaceticus

<220>
<221> CDS

<222> (1)..(1389)

<400> 3

```
gat gtt cct cta act cca tct caa ttt gct aaa gcg aaa tca gag aac      48
Asp Val Pro Leu Thr Pro Ser Gln Phe Ala Lys Ala Lys Ser Glu Asn
1               5                   10                  15

ttt gac aag aaa gtt att cta tct aat cta aat aag ccg cat gct ttg      96
Phe Asp Lys Lys Val Ile Leu Ser Asn Leu Asn Lys Pro His Ala Leu
            20                  25                  30

tta tgg gga cca gat aat caa att tgg tta act gag cga gca aca ggt     144
Leu Trp Gly Pro Asp Asn Gln Ile Trp Leu Thr Glu Arg Ala Thr Gly
        35                  40                  45

aag att cta aga gtt aat cca gag tcg ggt agt gta aaa aca gtt ttt     192
Lys Ile Leu Arg Val Asn Pro Glu Ser Gly Ser Val Lys Thr Val Phe
    50                  55                  60

cag gta cca gag att gtc aat gat gct gat ggg cag aat ggt tta tta     240
Gln Val Pro Glu Ile Val Asn Asp Ala Asp Gly Gln Asn Gly Leu Leu
65                  70                  75                  80

ggt ttt gcc ttc cat cct gat ttt aaa aat aat cct tat atc tat att     288
Gly Phe Ala Phe His Pro Asp Phe Lys Asn Asn Pro Tyr Ile Tyr Ile
                85                  90                  95

tca ggt aca ttt aaa aat ccg aaa tct aca gat aaa gaa tta ccg aac     336
Ser Gly Thr Phe Lys Asn Pro Lys Ser Thr Asp Lys Glu Leu Pro Asn
            100                 105                 110

caa acg att att cgt cgt tat acc tat aat aaa tca aca gat acg ctc     384
Gln Thr Ile Ile Arg Arg Tyr Thr Tyr Asn Lys Ser Thr Asp Thr Leu
        115                 120                 125

gag aag cca gtc gat tta tta gca gga tta cct tca tca aaa gac cat     432
Glu Lys Pro Val Asp Leu Leu Ala Gly Leu Pro Ser Ser Lys Asp His
    130                 135                 140

cag tca ggt cgt ctt gtc att ggg cca gat caa aag att tat tat acg     480
Gln Ser Gly Arg Leu Val Ile Gly Pro Asp Gln Lys Ile Tyr Tyr Thr
145                 150                 155                 160

att ggt gac caa ggg cgt aac cag ctt gct tat ttg ttc ttg cca aat     528
Ile Gly Asp Gln Gly Arg Asn Gln Leu Ala Tyr Leu Phe Leu Pro Asn
                165                 170                 175

caa gca caa cat acg cca act caa caa gaa ctg aat ggt aaa gac tat     576
Gln Ala Gln His Thr Pro Thr Gln Gln Glu Leu Asn Gly Lys Asp Tyr
            180                 185                 190

cac acc tat atg ggt aaa gta cta cgc tta aat ctt gat gga agt att     624
His Thr Tyr Met Gly Lys Val Leu Arg Leu Asn Leu Asp Gly Ser Ile
        195                 200                 205

cca aag gat aat cca agt ttt aac ggg gtg gtt agc cat att tat aca     672
Pro Lys Asp Asn Pro Ser Phe Asn Gly Val Val Ser His Ile Tyr Thr
    210                 215                 220

ctt gga cat cgt aat ccg cag ggc tta gca ttc act cca aat ggt aaa     720
Leu Gly His Arg Asn Pro Gln Gly Leu Ala Phe Thr Pro Asn Gly Lys
225                 230                 235                 240

tta ttg cag tct gaa caa ggc cca aac tct gac gat gaa att aac ctc     768
Leu Leu Gln Ser Glu Gln Gly Pro Asn Ser Asp Asp Glu Ile Asn Leu
                245                 250                 255
```

```
att gtc aaa ggt ggc aat tat ggt tgg ccg aat gta gca ggt tat aaa      816
Ile Val Lys Gly Gly Asn Tyr Gly Trp Pro Asn Val Ala Gly Tyr Lys
            260                 265                 270

gat gat agt ggc tat gct tat gca aat tat tca gca gca gcc aat aag      864
Asp Asp Ser Gly Tyr Ala Tyr Ala Asn Tyr Ser Ala Ala Ala Asn Lys
        275                 280                 285

tca att aag gat tta gct caa aat gga gta aaa gta gcc gca ggg gtc      912
Ser Ile Lys Asp Leu Ala Gln Asn Gly Val Lys Val Ala Ala Gly Val
    290                 295                 300

cct gtg acg aaa gaa tct gaa tgg act ggt aaa aac ttt gtc cca cca      960
Pro Val Thr Lys Glu Ser Glu Trp Thr Gly Lys Asn Phe Val Pro Pro
305                 310                 315                     320

tta aaa act tta tat acc gtt caa gat acc tac aac tat aac gat cca     1008
Leu Lys Thr Leu Tyr Thr Val Gln Asp Thr Tyr Asn Tyr Asn Asp Pro
                325                 330                 335

act tgt gga gag atg acc tac att tgc tgg cca aca gtt gca ccg tca     1056
Thr Cys Gly Glu Met Thr Tyr Ile Cys Trp Pro Thr Val Ala Pro Ser
            340                 345                 350

tct gcc tat gtc tat aag ggc ggt aaa aaa gca att act ggt tgg gaa     1104
Ser Ala Tyr Val Tyr Lys Gly Gly Lys Lys Ala Ile Thr Gly Trp Glu
        355                 360                 365

aat aca tta ttg gtt cca tct tta aaa cgt ggt gtc att ttc cgt att     1152
Asn Thr Leu Leu Val Pro Ser Leu Lys Arg Gly Val Ile Phe Arg Ile
    370                 375                 380

aag tta gat cca act tat agc act act tat gat gac gct gta ccg atg     1200
Lys Leu Asp Pro Thr Tyr Ser Thr Thr Tyr Asp Asp Ala Val Pro Met
385                 390                 395                     400

ttt aag agc aac aac cgt tat cgt gat gtg att gca agt cca gat ggg     1248
Phe Lys Ser Asn Asn Arg Tyr Arg Asp Val Ile Ala Ser Pro Asp Gly
                405                 410                 415

aat gtc tta tat gta tta act gat act gcc gga tgt gtc caa aaa gat     1296
Asn Val Leu Tyr Val Leu Thr Asp Thr Ala Gly Cys Val Gln Lys Asp
            420                 425                 430

gat ggc tca gta aca aat aca tta gaa aac cca gga tct ctc att aag     1344
Asp Gly Ser Val Thr Asn Thr Leu Glu Asn Pro Gly Ser Leu Ile Lys
        435                 440                 445

ttc acc tat aag gct aag taa tac agt cgc att aaa aaa ccg atc         1389
Phe Thr Tyr Lys Ala Lys     Tyr Ser Arg Ile Lys Lys Pro Ile
    450                     455                 460
```

<210> 4
<211> 454
<212> PRT
<213> Acinetobacter calcoaceticus

<400> 4

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30
```

```
Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ser Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Asn Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Val Ser His Ile Tyr Thr
    210                 215                 220

Leu Gly His Arg Asn Pro Gln Gly Leu Ala Phe Thr Pro Asn Gly Lys
225                 230                 235                 240

Leu Leu Gln Ser Glu Gln Gly Pro Asn Ser Asp Asp Glu Ile Asn Leu
                245                 250                 255

Ile Val Lys Gly Gly Asn Tyr Gly Trp Pro Asn Val Ala Gly Tyr Lys
            260                 265                 270

Asp Asp Ser Gly Tyr Ala Tyr Ala Asn Tyr Ser Ala Ala Ala Asn Lys
        275                 280                 285

Ser Ile Lys Asp Leu Ala Gln Asn Gly Val Lys Val Ala Ala Gly Val
    290                 295                 300
```

```
Pro Val Thr Lys Glu Ser Glu Trp Thr Gly Lys Asn Phe Val Pro Pro
305                 310                 315                 320

Leu Lys Thr Leu Tyr Thr Val Gln Asp Thr Tyr Asn Tyr Asn Asp Pro
                325                 330                 335

Thr Cys Gly Glu Met Thr Tyr Ile Cys Trp Pro Thr Val Ala Pro Ser
            340                 345                 350

Ser Ala Tyr Val Tyr Lys Gly Gly Lys Lys Ala Ile Thr Gly Trp Glu
        355                 360                 365

Asn Thr Leu Leu Val Pro Ser Leu Lys Arg Gly Val Ile Phe Arg Ile
    370                 375                 380

Lys Leu Asp Pro Thr Tyr Ser Thr Thr Tyr Asp Asp Ala Val Pro Met
385                 390                 395                 400

Phe Lys Ser Asn Asn Arg Tyr Arg Asp Val Ile Ala Ser Pro Asp Gly
                405                 410                 415

Asn Val Leu Tyr Val Leu Thr Asp Thr Ala Gly Cys Val Gln Lys Asp
            420                 425                 430

Asp Gly Ser Val Thr Asn Thr Leu Glu Asn Pro Gly Ser Leu Ile Lys
        435                 440                 445

Phe Thr Tyr Lys Ala Lys
    450
```

<210> 5
<211> 1389
<212> DNA
<213> Acinetobacter calcoaceticus

<220>
<221> CDS
<222> (1)..(1389)

<400> 5

```
gat gtt cct cta act cca tct caa ttt gct aaa gcg aaa tca gag aac       48
Asp Val Pro Leu Thr Pro Ser Gln Phe Ala Lys Ala Lys Ser Glu Asn
1               5                   10                  15

ttt gac aag aaa gtt att cta tct aat cta aat aag ccg cat gct ttg       96
Phe Asp Lys Lys Val Ile Leu Ser Asn Leu Asn Lys Pro His Ala Leu
            20                  25                  30

tta tgg gga cca gat aat caa att tgg tta act gag cga gca aca ggt      144
Leu Trp Gly Pro Asp Asn Gln Ile Trp Leu Thr Glu Arg Ala Thr Gly
        35                  40                  45

aag att cta aga gtt aat cca gag tcg ggt agt gta aaa aca gtt ttt      192
Lys Ile Leu Arg Val Asn Pro Glu Ser Gly Ser Val Lys Thr Val Phe
    50                  55                  60
```

```
cag gta cca gag att gtc aat gat gct gat ggg cag aat ggt tta tta      240
Gln Val Pro Glu Ile Val Asn Asp Ala Asp Gly Gln Asn Gly Leu Leu
65                  70                  75                  80

ggt ttt gcc ttc cat cct gat ttt aaa aat aat cct tat atc tat att      288
Gly Phe Ala Phe His Pro Asp Phe Lys Asn Asn Pro Tyr Ile Tyr Ile
                85                  90                  95

tca ggt aca ttt aaa aat ccg aaa tct aca gat aaa gaa tta ccg aac      336
Ser Gly Thr Phe Lys Asn Pro Lys Ser Thr Asp Lys Glu Leu Pro Asn
            100                 105                 110

caa acg att att cgt cgt tat acc tat aat aaa tca aca gat acg ctc      384
Gln Thr Ile Ile Arg Arg Tyr Thr Tyr Asn Lys Ser Thr Asp Thr Leu
        115                 120                 125

gag aag cca gtc gat tta tta gca gga tta cct tca tca aaa gac cat      432
Glu Lys Pro Val Asp Leu Leu Ala Gly Leu Pro Ser Ser Lys Asp His
    130                 135                 140

cag tca ggt cgt ctt gtc att ggg cca gat caa aag att tat tat acg      480
Gln Ser Gly Arg Leu Val Ile Gly Pro Asp Gln Lys Ile Tyr Tyr Thr
145                 150                 155                 160

att ggt gac caa ggg cgt aac cag ctt gct tat ttg ttc ttg cca aat      528
Ile Gly Asp Gln Gly Arg Asn Gln Leu Ala Tyr Leu Phe Leu Pro Asn
                165                 170                 175

caa gca caa cat acg cca act caa caa gaa ctg aat ggt aaa gac tat      576
Gln Ala Gln His Thr Pro Thr Gln Gln Glu Leu Asn Gly Lys Asp Tyr
            180                 185                 190

cac acc tat atg ggt aaa gta cta cgc tta aat ctt gat gga agt att      624
His Thr Tyr Met Gly Lys Val Leu Arg Leu Asn Leu Asp Gly Ser Ile
        195                 200                 205

cca aag gat aat cca agt ttt aac ggg gtg gtt agc cat att tat aca      672
Pro Lys Asp Asn Pro Ser Phe Asn Gly Val Val Ser His Ile Tyr Thr
    210                 215                 220

ctt gga cat cgt aat ccg cag ggc tta gca ttc act cca aat ggt aaa      720
Leu Gly His Arg Asn Pro Gln Gly Leu Ala Phe Thr Pro Asn Gly Lys
225                 230                 235                 240

tta ttg cag tct gaa caa ggc cca aac tct gac gat gaa att aac ctc      768
Leu Leu Gln Ser Glu Gln Gly Pro Asn Ser Asp Asp Glu Ile Asn Leu
                245                 250                 255

att gtc aaa ggt ggc aat tat ggt tgg ccg aat gta gca ggt tat aaa      816
Ile Val Lys Gly Gly Asn Tyr Gly Trp Pro Asn Val Ala Gly Tyr Lys
            260                 265                 270

gat gat agt ggc tat gct tat gca aat tat tca gca gca gcc aat aag      864
Asp Asp Ser Gly Tyr Ala Tyr Ala Asn Tyr Ser Ala Ala Ala Asn Lys
        275                 280                 285

tca att aag gat tta gct caa aat gga gta aaa gta gcc gca ggg gtc      912
Ser Ile Lys Asp Leu Ala Gln Asn Gly Val Lys Val Ala Ala Gly Val
    290                 295                 300

cct gtg acg aaa gaa tct gaa tgg act ggt aaa aac ttt gtc cca cca      960
Pro Val Thr Lys Glu Ser Glu Trp Thr Gly Lys Asn Phe Val Pro Pro
305                 310                 315                 320

tta aaa act tta tat acc gtt caa gat acc tac aac tat aac gat cca     1008
Leu Lys Thr Leu Tyr Thr Val Gln Asp Thr Tyr Asn Tyr Asn Asp Pro
                325                 330                 335
```

```
act tgt gga gag atg acc tac att tgc tgg cca aca gtt gca ccg tca      1056
Thr Cys Gly Glu Met Thr Tyr Ile Cys Trp Pro Thr Val Ala Pro Ser
            340                 345                 350

tct gcc tat gtc tat aag ggc ggt aaa aaa gca att act ggt tgg gaa      1104
Ser Ala Tyr Val Tyr Lys Gly Gly Lys Lys Ala Ile Thr Gly Trp Glu
        355                 360                 365

aat aca tta ttg gtt cca tct tta aaa cgt ggt gtc att ttc cgt att      1152
Asn Thr Leu Leu Val Pro Ser Leu Lys Arg Gly Val Ile Phe Arg Ile
    370                 375                 380

aag tta gat cca act tat agc act act tat gat gac gct gta ccg atg      1200
Lys Leu Asp Pro Thr Tyr Ser Thr Thr Tyr Asp Asp Ala Val Pro Met
385                 390                 395                 400

ttt aag agc aac aac cgt tat cgt gat gtg att gca agt cca gat ggg      1248
Phe Lys Ser Asn Asn Arg Tyr Arg Asp Val Ile Ala Ser Pro Asp Gly
                405                 410                 415

aat gtc tta tat gta tta act gat act gcc gga tat gtc caa aaa gat      1296
Asn Val Leu Tyr Val Leu Thr Asp Thr Ala Gly Tyr Val Gln Lys Asp
            420                 425                 430

gat ggc tca gta aca aat aca tta gaa aac cca gga tct ctc att aag      1344
Asp Gly Ser Val Thr Asn Thr Leu Glu Asn Pro Gly Ser Leu Ile Lys
        435                 440                 445

ttc acc tat aag gct aag taa tac agt cgc att aaa aaa ccg atc          1389
Phe Thr Tyr Lys Ala Lys     Tyr Ser Arg Ile Lys Lys Pro Ile
    450                     455                 460
```

<210> 6
<211> 454
<212> PRT
<213> Acinetobacter calcoaceticus

<400> 6

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110
```

Gln Thr Ile Ile Arg Arg Tyr Thr Tyr Asn Lys Ser Thr Asp Thr Leu
115 120 125

Glu Lys Pro Val Asp Leu Leu Ala Gly Leu Pro Ser Ser Lys Asp His
130 135 140

Gln Ser Gly Arg Leu Val Ile Gly Pro Asp Gln Lys Ile Tyr Tyr Thr
145 150 155 160

Ile Gly Asp Gln Gly Arg Asn Gln Leu Ala Tyr Leu Phe Leu Pro Asn
165 170 175

Gln Ala Gln His Thr Pro Thr Gln Gln Glu Leu Asn Gly Lys Asp Tyr
180 185 190

His Thr Tyr Met Gly Lys Val Leu Arg Leu Asn Leu Asp Gly Ser Ile
195 200 205

Pro Lys Asp Asn Pro Ser Phe Asn Gly Val Val Ser His Ile Tyr Thr
210 215 220

Leu Gly His Arg Asn Pro Gln Gly Leu Ala Phe Thr Pro Asn Gly Lys
225 230 235 240

Leu Leu Gln Ser Glu Gln Gly Pro Asn Ser Asp Asp Glu Ile Asn Leu
245 250 255

Ile Val Lys Gly Gly Asn Tyr Gly Trp Pro Asn Val Ala Gly Tyr Lys
260 265 270

Asp Asp Ser Gly Tyr Ala Tyr Ala Asn Tyr Ser Ala Ala Ala Asn Lys
275 280 285

Ser Ile Lys Asp Leu Ala Gln Asn Gly Val Lys Val Ala Ala Gly Val
290 295 300

Pro Val Thr Lys Glu Ser Glu Trp Thr Gly Lys Asn Phe Val Pro Pro
305 310 315 320

Leu Lys Thr Leu Tyr Thr Val Gln Asp Thr Tyr Asn Tyr Asn Asp Pro
325 330 335

Thr Cys Gly Glu Met Thr Tyr Ile Cys Trp Pro Thr Val Ala Pro Ser
340 345 350

Ser Ala Tyr Val Tyr Lys Gly Gly Lys Lys Ala Ile Thr Gly Trp Glu
355 360 365

Asn Thr Leu Leu Val Pro Ser Leu Lys Arg Gly Val Ile Phe Arg Ile
370 375 380

```
Lys Leu Asp Pro Thr Tyr Ser Thr Thr Tyr Asp Asp Ala Val Pro Met
385                 390                 395                 400

Phe Lys Ser Asn Asn Arg Tyr Arg Asp Val Ile Ala Ser Pro Asp Gly
                405                 410                 415

Asn Val Leu Tyr Val Leu Thr Asp Thr Ala Gly Tyr Val Gln Lys Asp
            420                 425                 430

Asp Gly Ser Val Thr Asn Thr Leu Glu Asn Pro Gly Ser Leu Ile Lys
        435                 440                 445

Phe Thr Tyr Lys Ala Lys
    450
```

<210> 7
<211> 1389
<212> DNA
<213> Acinetobacter calcoaceticus

<220>
<221> CDS
<222> (1)..(1389)

<400> 7

```
gat gtt cct cta act cca tct caa ttt gct aaa gcg aaa tca gag aac      48
Asp Val Pro Leu Thr Pro Ser Gln Phe Ala Lys Ala Lys Ser Glu Asn
1               5                   10                  15

ttt gac aag aaa gtt att cta tct aat cta aat aag ccg cat gct ttg      96
Phe Asp Lys Lys Val Ile Leu Ser Asn Leu Asn Lys Pro His Ala Leu
            20                  25                  30

tta tgg gga cca gat aat caa att tgg tta act gag cga gca aca ggt     144
Leu Trp Gly Pro Asp Asn Gln Ile Trp Leu Thr Glu Arg Ala Thr Gly
        35                  40                  45

aag att cta aga gtt aat cca gag tcg ggt agt gta aaa aca gtt ttt     192
Lys Ile Leu Arg Val Asn Pro Glu Ser Gly Ser Val Lys Thr Val Phe
    50                  55                  60

cag gta cca gag att gtc aat gat gct gat ggg cag aat ggt tta tta     240
Gln Val Pro Glu Ile Val Asn Asp Ala Asp Gly Gln Asn Gly Leu Leu
65                  70                  75                  80

ggt ttt gcc ttc cat cct gat ttt aaa aat aat cct tat atc tat att     288
Gly Phe Ala Phe His Pro Asp Phe Lys Asn Asn Pro Tyr Ile Tyr Ile
                85                  90                  95

tca ggt aca ttt aaa aat ccg aaa tct aca gat aaa gaa tta ccg aac     336
Ser Gly Thr Phe Lys Asn Pro Lys Ser Thr Asp Lys Glu Leu Pro Asn
            100                 105                 110

caa acg att att cgt cgt tat acc tat aat aaa tca aca gat acg ctc     384
Gln Thr Ile Ile Arg Arg Tyr Thr Tyr Asn Lys Ser Thr Asp Thr Leu
        115                 120                 125

gag aag cca gtc gat tta tta gca gga tta cct tca tca aaa gac cat     432
Glu Lys Pro Val Asp Leu Leu Ala Gly Leu Pro Ser Ser Lys Asp His
    130                 135                 140
```

```
cag tca ggt cgt ctt gtc att ggg cca gat caa aag att tat tat acg        480
Gln Ser Gly Arg Leu Val Ile Gly Pro Asp Gln Lys Ile Tyr Tyr Thr
145                 150                 155                 160

att ggt gac caa ggg cgt aac cag ctt gct tat ttg ttc ttg cca aat        528
Ile Gly Asp Gln Gly Arg Asn Gln Leu Ala Tyr Leu Phe Leu Pro Asn
                165                 170                 175

caa gca caa cat acg cca act caa caa gaa ctg aat ggt aaa gac tat        576
Gln Ala Gln His Thr Pro Thr Gln Gln Glu Leu Asn Gly Lys Asp Tyr
            180                 185                 190

cac acc tat atg ggt aaa gta cta cgc tta aat ctt gat gga agt att        624
His Thr Tyr Met Gly Lys Val Leu Arg Leu Asn Leu Asp Gly Ser Ile
        195                 200                 205

cca aag gat aat cca agt ttt aac ggg gtg gtt agc cat att tat aca        672
Pro Lys Asp Asn Pro Ser Phe Asn Gly Val Val Ser His Ile Tyr Thr
    210                 215                 220

ctt gga cat cgt aat ccg cag ggc tta gca ttc act cca aat ggt aaa        720
Leu Gly His Arg Asn Pro Gln Gly Leu Ala Phe Thr Pro Asn Gly Lys
225                 230                 235                 240

tta ttg cag tct gaa caa ggc cca aac tct gac gat gaa att aac ctc        768
Leu Leu Gln Ser Glu Gln Gly Pro Asn Ser Asp Asp Glu Ile Asn Leu
                245                 250                 255

att gtc aaa ggt ggc aat tat ggt tgg ccg aat gta gca ggt tat aaa        816
Ile Val Lys Gly Gly Asn Tyr Gly Trp Pro Asn Val Ala Gly Tyr Lys
            260                 265                 270

gat gat agt ggc tat gct tat gca aat tat tca gca gca gcc aat aag        864
Asp Asp Ser Gly Tyr Ala Tyr Ala Asn Tyr Ser Ala Ala Ala Asn Lys
        275                 280                 285

tca att aag gat tta gct caa aat gga gta aaa gta gcc gca ggg gtc        912
Ser Ile Lys Asp Leu Ala Gln Asn Gly Val Lys Val Ala Ala Gly Val
    290                 295                 300

cct gtg acg aaa gaa tct gaa tgg act ggt aaa aac ttt gtc cca cca        960
Pro Val Thr Lys Glu Ser Glu Trp Thr Gly Lys Asn Phe Val Pro Pro
305                 310                 315                 320

tta aaa act tta tat acc gtt caa gat acc tac aac tat aac gat cca       1008
Leu Lys Thr Leu Tyr Thr Val Gln Asp Thr Tyr Asn Tyr Asn Asp Pro
                325                 330                 335

act tgt gga gag atg acc tac att tgc tgg cca aca gtt gca ccg tca       1056
Thr Cys Gly Glu Met Thr Tyr Ile Cys Trp Pro Thr Val Ala Pro Ser
            340                 345                 350

tct gcc tat gtc tat aag ggc ggt aaa aaa gca att act ggt tgg gaa       1104
Ser Ala Tyr Val Tyr Lys Gly Gly Lys Lys Ala Ile Thr Gly Trp Glu
        355                 360                 365

aat aca tta ttg gtt cca tct tta aaa cgt ggt gtc att ttc cgt att       1152
Asn Thr Leu Leu Val Pro Ser Leu Lys Arg Gly Val Ile Phe Arg Ile
    370                 375                 380

aag tta gat cca act tat agc act act tat gat gac gct gta ccg atg       1200
Lys Leu Asp Pro Thr Tyr Ser Thr Thr Tyr Asp Asp Ala Val Pro Met
385                 390                 395                 400

ttt aag agc aac aac cgt tat cgt gat gtg att gca agt cca gat ggg       1248
Phe Lys Ser Asn Asn Arg Tyr Arg Asp Val Ile Ala Ser Pro Asp Gly
                405                 410                 415
```

```
aat gtc tta tat gta tta act gat act gcc gga gct gtc caa aaa gat      1296
Asn Val Leu Tyr Val Leu Thr Asp Thr Ala Gly Ala Val Gln Lys Asp
            420                 425                 430

gat ggc tca gta aca aat aca tta gaa aac cca gga tct ctc att aag      1344
Asp Gly Ser Val Thr Asn Thr Leu Glu Asn Pro Gly Ser Leu Ile Lys
        435                 440                 445

ttc acc tat aag gct aag taa tac agt cgc att aaa aaa ccg atc          1389
Phe Thr Tyr Lys Ala Lys     Tyr Ser Arg Ile Lys Lys Pro Ile
    450                     455                 460
```

<210> 8
<211> 454
<212> PRT
<213> Acinetobacter calcoaceticus

<400> 8

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ser Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Asn Gly Lys Asp Tyr
            180                 185                 190
```

His Thr Tyr Met Gly Lys Val Leu Arg Leu Asn Leu Asp Gly Ser Ile
195 200 205

Pro Lys Asp Asn Pro Ser Phe Asn Gly Val Val Ser His Ile Tyr Thr
210 215 220

Leu Gly His Arg Asn Pro Gln Gly Leu Ala Phe Thr Pro Asn Gly Lys
225 230 235 240

Leu Leu Gln Ser Glu Gln Gly Pro Asn Ser Asp Asp Glu Ile Asn Leu
245 250 255

Ile Val Lys Gly Gly Asn Tyr Gly Trp Pro Asn Val Ala Gly Tyr Lys
260 265 270

Asp Asp Ser Gly Tyr Ala Tyr Ala Asn Tyr Ser Ala Ala Ala Asn Lys
275 280 285

Ser Ile Lys Asp Leu Ala Gln Asn Gly Val Lys Val Ala Ala Gly Val
290 295 300

Pro Val Thr Lys Glu Ser Glu Trp Thr Gly Lys Asn Phe Val Pro Pro
305 310 315 320

Leu Lys Thr Leu Tyr Thr Val Gln Asp Thr Tyr Asn Tyr Asn Asp Pro
325 330 335

Thr Cys Gly Glu Met Thr Tyr Ile Cys Trp Pro Thr Val Ala Pro Ser
340 345 350

Ser Ala Tyr Val Tyr Lys Gly Gly Lys Lys Ala Ile Thr Gly Trp Glu
355 360 365

Asn Thr Leu Leu Val Pro Ser Leu Lys Arg Gly Val Ile Phe Arg Ile
370 375 380

Lys Leu Asp Pro Thr Tyr Ser Thr Thr Tyr Asp Asp Ala Val Pro Met
385 390 395 400

Phe Lys Ser Asn Asn Arg Tyr Arg Asp Val Ile Ala Ser Pro Asp Gly
405 410 415

Asn Val Leu Tyr Val Leu Thr Asp Thr Ala Gly Ala Val Gln Lys Asp
420 425 430

Asp Gly Ser Val Thr Asn Thr Leu Glu Asn Pro Gly Ser Leu Ile Lys
435 440 445

Phe Thr Tyr Lys Ala Lys
450

<210> 9
<211> 1389
<212> DNA

<213> Acinetobacter calcoaceticus

<220>
<221> CDS
<222> (1)..(1389)

<400> 9

```
gat gtt cct cta act cca tct caa ttt gct aaa gcg aaa tca gag aac      48
Asp Val Pro Leu Thr Pro Ser Gln Phe Ala Lys Ala Lys Ser Glu Asn
1               5                   10                  15

ttt gac aag aaa gtt att cta tct aat cta aat aag ccg cat gct ttg      96
Phe Asp Lys Lys Val Ile Leu Ser Asn Leu Asn Lys Pro His Ala Leu
            20                  25                  30

tta tgg gga cca gat aat caa att tgg tta act gag cga gca aca ggt      144
Leu Trp Gly Pro Asp Asn Gln Ile Trp Leu Thr Glu Arg Ala Thr Gly
        35                  40                  45

aag att cta aga gtt aat cca gag tcg ggt agt gta aaa aca gtt ttt      192
Lys Ile Leu Arg Val Asn Pro Glu Ser Gly Ser Val Lys Thr Val Phe
    50                  55                  60

cag gta cca gag att gtc aat gat gct gat ggg cag aat ggt tta tta      240
Gln Val Pro Glu Ile Val Asn Asp Ala Asp Gly Gln Asn Gly Leu Leu
65                  70                  75                  80

ggt ttt gcc ttc cat cct gat ttt aaa aat aat cct tat atc tat att      288
Gly Phe Ala Phe His Pro Asp Phe Lys Asn Asn Pro Tyr Ile Tyr Ile
                85                  90                  95

tca ggt aca ttt aaa aat ccg aaa tct aca gat aaa gaa tta ccg aac      336
Ser Gly Thr Phe Lys Asn Pro Lys Ser Thr Asp Lys Glu Leu Pro Asn
            100                 105                 110

caa acg att att cgt cgt tat acc tat aat aaa tca aca gat acg ctc      384
Gln Thr Ile Ile Arg Arg Tyr Thr Tyr Asn Lys Ser Thr Asp Thr Leu
        115                 120                 125

gag aag cca gtc gat tta tta gca gga tta cct tca tca aaa gac cat      432
Glu Lys Pro Val Asp Leu Leu Ala Gly Leu Pro Ser Ser Lys Asp His
    130                 135                 140

cag tca ggt cgt ctt gtc att ggg cca gat caa aag att tat tat acg      480
Gln Ser Gly Arg Leu Val Ile Gly Pro Asp Gln Lys Ile Tyr Tyr Thr
145                 150                 155                 160

att ggt gac caa ggg cgt aac cag ctt gct tat ttg ttc ttg cca aat      528
Ile Gly Asp Gln Gly Arg Asn Gln Leu Ala Tyr Leu Phe Leu Pro Asn
                165                 170                 175

caa gca caa cat acg cca act caa caa gaa ctg aat ggt aaa gac tat      576
Gln Ala Gln His Thr Pro Thr Gln Gln Glu Leu Asn Gly Lys Asp Tyr
            180                 185                 190

cac acc tat atg ggt aaa gta cta cgc tta aat ctt gat gga agt att      624
His Thr Tyr Met Gly Lys Val Leu Arg Leu Asn Leu Asp Gly Ser Ile
        195                 200                 205

cca aag gat aat cca agt ttt aac ggg gtg gtt agc cat att tat aca      672
Pro Lys Asp Asn Pro Ser Phe Asn Gly Val Val Ser His Ile Tyr Thr
```

```
    210                     215                     220

ctt gga cat cgt aat ccg cag ggc tta gca ttc act cca aat ggt aaa      720
Leu Gly His Arg Asn Pro Gln Gly Leu Ala Phe Thr Pro Asn Gly Lys
225                     230                     235                 240

tta ttg cag tct gaa caa ggc cca aac tct gac gat gaa att aac ctc      768
Leu Leu Gln Ser Glu Gln Gly Pro Asn Ser Asp Asp Glu Ile Asn Leu
                245                     250                     255

att gtc aaa ggt ggc aat tat ggt tgg ccg aat gta gca ggt tat aaa      816
Ile Val Lys Gly Gly Asn Tyr Gly Trp Pro Asn Val Ala Gly Tyr Lys
            260                     265                     270

gat gat agt ggc tat gct tat gca aat tat tca gca gca gcc aat aag      864
Asp Asp Ser Gly Tyr Ala Tyr Ala Asn Tyr Ser Ala Ala Ala Asn Lys
        275                     280                     285

tca att aag gat tta gct caa aat gga gta aaa gta gcc gca ggg gtc      912
Ser Ile Lys Asp Leu Ala Gln Asn Gly Val Lys Val Ala Ala Gly Val
    290                     295                     300

cct gtg acg aaa gaa tct gaa tgg act ggt aaa aac ttt gtc cca cca      960
Pro Val Thr Lys Glu Ser Glu Trp Thr Gly Lys Asn Phe Val Pro Pro
305                     310                     315                 320

tta aaa act tta tat acc gtt caa gat acc tac aac tat aac gat cca     1008
Leu Lys Thr Leu Tyr Thr Val Gln Asp Thr Tyr Asn Tyr Asn Asp Pro
                325                     330                     335

act tgt gga gag atg acc tac att tgc tgg cca aca gtt gca ccg tca     1056
Thr Cys Gly Glu Met Thr Tyr Ile Cys Trp Pro Thr Val Ala Pro Ser
            340                     345                     350

tct gcc tat gtc tat aag ggc ggt aaa aaa gca att act ggt tgg gaa     1104
Ser Ala Tyr Val Tyr Lys Gly Gly Lys Lys Ala Ile Thr Gly Trp Glu
        355                     360                     365

aat aca tta ttg gtt cca tct tta aaa cgt ggt gtc att ttc cgt att     1152
Asn Thr Leu Leu Val Pro Ser Leu Lys Arg Gly Val Ile Phe Arg Ile
    370                     375                     380

aag tta gat cca act tat agc act act tat gat gac gct gta ccg atg     1200
Lys Leu Asp Pro Thr Tyr Ser Thr Thr Tyr Asp Asp Ala Val Pro Met
385                     390                     395                 400

ttt aag agc aac aac cgt tat cgt gat gtg att gca agt cca gat ggg     1248
Phe Lys Ser Asn Asn Arg Tyr Arg Asp Val Ile Ala Ser Pro Asp Gly
                405                     410                     415

aat gtc tta tat gta tta act gat act gcc gga aag gtc caa aaa gat     1296
Asn Val Leu Tyr Val Leu Thr Asp Thr Ala Gly Lys Val Gln Lys Asp
            420                     425                     430

gat ggc tca gta aca aat aca tta gaa aac cca gga tct ctc att aag     1344
Asp Gly Ser Val Thr Asn Thr Leu Glu Asn Pro Gly Ser Leu Ile Lys
        435                     440                     445

ttc acc tat aag gct aag taa tac agt cgc att aaa aaa ccg atc         1389
Phe Thr Tyr Lys Ala Lys     Tyr Ser Arg Ile Lys Lys Pro Ile
    450                     455                     460
```

<210> 10

<211> 454

<212> PRT

<213> Acinetobacter calcoaceticus

<400> 10

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ser Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Asn Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Val Ser His Ile Tyr Thr
    210                 215                 220

Leu Gly His Arg Asn Pro Gln Gly Leu Ala Phe Thr Pro Asn Gly Lys
225                 230                 235                 240

Leu Leu Gln Ser Glu Gln Gly Pro Asn Ser Asp Asp Glu Ile Asn Leu
                245                 250                 255

Ile Val Lys Gly Gly Asn Tyr Gly Trp Pro Asn Val Ala Gly Tyr Lys
            260                 265                 270
```

```
Asp Asp Ser Gly Tyr Ala Tyr Ala Asn Tyr Ser Ala Ala Ala Asn Lys
        275                 280                 285

Ser Ile Lys Asp Leu Ala Gln Asn Gly Val Lys Val Ala Ala Gly Val
    290                 295                 300

Pro Val Thr Lys Glu Ser Glu Trp Thr Gly Lys Asn Phe Val Pro Pro
305                 310                 315                 320

Leu Lys Thr Leu Tyr Thr Val Gln Asp Thr Tyr Asn Tyr Asn Asp Pro
                325                 330                 335

Thr Cys Gly Glu Met Thr Tyr Ile Cys Trp Pro Thr Val Ala Pro Ser
            340                 345                 350

Ser Ala Tyr Val Tyr Lys Gly Gly Lys Lys Ala Ile Thr Gly Trp Glu
        355                 360                 365

Asn Thr Leu Leu Val Pro Ser Leu Lys Arg Gly Val Ile Phe Arg Ile
    370                 375                 380

Lys Leu Asp Pro Thr Tyr Ser Thr Thr Tyr Asp Asp Ala Val Pro Met
385                 390                 395                 400

Phe Lys Ser Asn Asn Arg Tyr Arg Asp Val Ile Ala Ser Pro Asp Gly
                405                 410                 415

Asn Val Leu Tyr Val Leu Thr Asp Thr Ala Gly Lys Val Gln Lys Asp
            420                 425                 430

Asp Gly Ser Val Thr Asn Thr Leu Glu Asn Pro Gly Ser Leu Ile Lys
        435                 440                 445

Phe Thr Tyr Lys Ala Lys
    450
```

<210> 11
<211> 1389
<212> DNA
<213> Acinetobacter calcoaceticus

<220>
<221> CDS
<222> (1)..(1389)

<400> 11

```
gat gtt cct cta act cca tct caa ttt gct aaa gcg aaa tca gag aac       48
Asp Val Pro Leu Thr Pro Ser Gln Phe Ala Lys Ala Lys Ser Glu Asn
1               5                   10                  15

ttt gac aag aaa gtt att cta tct aat cta aat aag ccg cat gct ttg       96
Phe Asp Lys Lys Val Ile Leu Ser Asn Leu Asn Lys Pro His Ala Leu
```

```
                    20                       25                       30

tta tgg gga cca gat aat caa att tgg tta act gag cga gca aca ggt      144
Leu Trp Gly Pro Asp Asn Gln Ile Trp Leu Thr Glu Arg Ala Thr Gly
        35                  40                  45

aag att cta aga gtt aat cca gag tcg ggt agt gta aaa aca gtt ttt      192
Lys Ile Leu Arg Val Asn Pro Glu Ser Gly Ser Val Lys Thr Val Phe
    50                  55                  60

cag gta cca gag att gtc aat gat gct gat ggg cag aat ggt tta tta      240
Gln Val Pro Glu Ile Val Asn Asp Ala Asp Gly Gln Asn Gly Leu Leu
65                  70                  75                  80

ggt ttt gcc ttc cat cct gat ttt aaa aat aat cct tat atc tat att      288
Gly Phe Ala Phe His Pro Asp Phe Lys Asn Asn Pro Tyr Ile Tyr Ile
                85                  90                  95

tca ggt aca ttt aaa aat ccg aaa tct aca gat aaa gaa tta ccg aac      336
Ser Gly Thr Phe Lys Asn Pro Lys Ser Thr Asp Lys Glu Leu Pro Asn
            100                 105                 110

caa acg att att cgt cgt tat acc tat aat aaa tca aca gat acg ctc      384
Gln Thr Ile Ile Arg Arg Tyr Thr Tyr Asn Lys Ser Thr Asp Thr Leu
        115                 120                 125

gag aag cca gtc gat tta tta gca gga tta cct tca tca aaa gac cat      432
Glu Lys Pro Val Asp Leu Leu Ala Gly Leu Pro Ser Ser Lys Asp His
    130                 135                 140

cag tca ggt cgt ctt gtc att ggg cca gat caa aag att tat tat acg      480
Gln Ser Gly Arg Leu Val Ile Gly Pro Asp Gln Lys Ile Tyr Tyr Thr
145                 150                 155                 160

att ggt gac caa ggg cgt aac cag ctt gct tat ttg ttc ttg cca aat      528
Ile Gly Asp Gln Gly Arg Asn Gln Leu Ala Tyr Leu Phe Leu Pro Asn
                165                 170                 175

caa gca caa cat acg cca act caa caa gaa ctg aat ggt aaa gac tat      576
Gln Ala Gln His Thr Pro Thr Gln Gln Glu Leu Asn Gly Lys Asp Tyr
            180                 185                 190

cac acc tat atg ggt aaa gta cta cgc tta aat ctt gat gga agt att      624
His Thr Tyr Met Gly Lys Val Leu Arg Leu Asn Leu Asp Gly Ser Ile
        195                 200                 205

cca aag gat aat cca agt ttt aac ggg gtg gtt agc cat att tat aca      672
Pro Lys Asp Asn Pro Ser Phe Asn Gly Val Val Ser His Ile Tyr Thr
    210                 215                 220

ctt gga cat cgt aat ccg cag ggc tta gca ttc act cca aat ggt aaa      720
Leu Gly His Arg Asn Pro Gln Gly Leu Ala Phe Thr Pro Asn Gly Lys
225                 230                 235                 240

tta ttg cag tct gaa caa ggc cca aac tct gac gat gaa att aac ctc      768
Leu Leu Gln Ser Glu Gln Gly Pro Asn Ser Asp Asp Glu Ile Asn Leu
                245                 250                 255

att gtc aaa ggt ggc aat tat ggt tgg ccg aat gta gca ggt tat aaa      816
Ile Val Lys Gly Gly Asn Tyr Gly Trp Pro Asn Val Ala Gly Tyr Lys
            260                 265                 270

gat gat agt ggc tat gct tat gca aat tat tca gca gca gcc aat aag      864
Asp Asp Ser Gly Tyr Ala Tyr Ala Asn Tyr Ser Ala Ala Ala Asn Lys
        275                 280                 285

tca att aag gat tta gct caa aat gga gta aaa gta gcc gca ggg gtc      912
Ser Ile Lys Asp Leu Ala Gln Asn Gly Val Lys Val Ala Ala Gly Val
```

```
                290                     295                     300

cct gtg acg aaa gaa tct gaa tgg act ggt aaa aac ttt gtc cca cca      960
Pro Val Thr Lys Glu Ser Glu Trp Thr Gly Lys Asn Phe Val Pro Pro
305                 310                     315                 320

tta aaa act tta tat acc gtt caa gat acc tac aac tat aac gat cca      1008
Leu Lys Thr Leu Tyr Thr Val Gln Asp Thr Tyr Asn Tyr Asn Asp Pro
                325                 330                     335

act tgt gga gag atg acc tac att tgc tgg cca aca gtt gca ccg tca      1056
Thr Cys Gly Glu Met Thr Tyr Ile Cys Trp Pro Thr Val Ala Pro Ser
            340                 345                     350

tct gcc tat gtc tat aag ggc ggt aaa aaa gca att act ggt tgg gaa      1104
Ser Ala Tyr Val Tyr Lys Gly Gly Lys Lys Ala Ile Thr Gly Trp Glu
        355                 360                     365

aat aca tta ttg gtt cca tct tta aaa cgt ggt gtc att ttc cgt att      1152
Asn Thr Leu Leu Val Pro Ser Leu Lys Arg Gly Val Ile Phe Arg Ile
    370                 375                     380

aag tta gat cca act tat agc act act tat gat gac gct gta ccg atg      1200
Lys Leu Asp Pro Thr Tyr Ser Thr Thr Tyr Asp Asp Ala Val Pro Met
385                 390                     395                 400

ttt aag agc aac aac cgt tat cgt gat gtg att gca agt cca gat ggg      1248
Phe Lys Ser Asn Asn Arg Tyr Arg Asp Val Ile Ala Ser Pro Asp Gly
                405                 410                     415

aat gtc tta tat gta tta act gat act gcc gga gac gtc caa aaa gat      1296
Asn Val Leu Tyr Val Leu Thr Asp Thr Ala Gly Asp Val Gln Lys Asp
            420                 425                     430

gat ggc tca gta aca aat aca tta gaa aac cca gga tct ctc att aag      1344
Asp Gly Ser Val Thr Asn Thr Leu Glu Asn Pro Gly Ser Leu Ile Lys
        435                 440                     445

ttc acc tat aag gct aag taa tac agt cgc att aaa aaa ccg atc          1389
Phe Thr Tyr Lys Ala Lys     Tyr Ser Arg Ile Lys Lys Pro Ile
    450                     455                     460
```

<210> 12
<211> 454
<212> PRT
<213> Acinetobacter calcoaceticus

<400> 12

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80
```

```
Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ser Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Asn Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Val Ser His Ile Tyr Thr
    210                 215                 220

Leu Gly His Arg Asn Pro Gln Gly Leu Ala Phe Thr Pro Asn Gly Lys
225                 230                 235                 240

Leu Leu Gln Ser Glu Gln Gly Pro Asn Ser Asp Asp Glu Ile Asn Leu
                245                 250                 255

Ile Val Lys Gly Gly Asn Tyr Gly Trp Pro Asn Val Ala Gly Tyr Lys
            260                 265                 270

Asp Asp Ser Gly Tyr Ala Tyr Ala Asn Tyr Ser Ala Ala Ala Asn Lys
        275                 280                 285

Ser Ile Lys Asp Leu Ala Gln Asn Gly Val Lys Val Ala Ala Gly Val
    290                 295                 300

Pro Val Thr Lys Glu Ser Glu Trp Thr Gly Lys Asn Phe Val Pro Pro
305                 310                 315                 320

Leu Lys Thr Leu Tyr Thr Val Gln Asp Thr Tyr Asn Tyr Asn Asp Pro
                325                 330                 335

Thr Cys Gly Glu Met Thr Tyr Ile Cys Trp Pro Thr Val Ala Pro Ser
            340                 345                 350
```

```
Ser Ala Tyr Val Tyr Lys Gly Gly Lys Lys Ala Ile Thr Gly Trp Glu
        355                 360                 365

Asn Thr Leu Leu Val Pro Ser Leu Lys Arg Gly Val Ile Phe Arg Ile
    370                 375                 380

Lys Leu Asp Pro Thr Tyr Ser Thr Thr Tyr Asp Asp Ala Val Pro Met
385                 390                 395                 400

Phe Lys Ser Asn Asn Arg Tyr Arg Asp Val Ile Ala Ser Pro Asp Gly
                405                 410                 415

Asn Val Leu Tyr Val Leu Thr Asp Thr Ala Gly Asp Val Gln Lys Asp
            420                 425                 430

Asp Gly Ser Val Thr Asn Thr Leu Glu Asn Pro Gly Ser Leu Ile Lys
        435                 440                 445

Phe Thr Tyr Lys Ala Lys
    450
```

<210> 13
<211> 1389
<212> DNA
<213> Acinetobacter calcoaceticus

<220>
<221> CDS
<222> (1)..(1389)

<400> 13

```
gat gtt cct cta act cca tct caa ttt gct aaa gcg aaa tca gag aac      48
Asp Val Pro Leu Thr Pro Ser Gln Phe Ala Lys Ala Lys Ser Glu Asn
1               5                   10                  15

ttt gac aag aaa gtt att cta tct aat cta aat aag ccg cat gct ttg      96
Phe Asp Lys Lys Val Ile Leu Ser Asn Leu Asn Lys Pro His Ala Leu
            20                  25                  30

tta tgg gga cca gat aat caa att tgg tta act gag cga gca aca ggt     144
Leu Trp Gly Pro Asp Asn Gln Ile Trp Leu Thr Glu Arg Ala Thr Gly
        35                  40                  45

aag att cta aga gtt aat cca gag tcg ggt agt gta aaa aca gtt ttt     192
Lys Ile Leu Arg Val Asn Pro Glu Ser Gly Ser Val Lys Thr Val Phe
    50                  55                  60

cag gta cca gag att gtc aat gat gct gat ggg cag aat ggt tta tta     240
Gln Val Pro Glu Ile Val Asn Asp Ala Asp Gly Gln Asn Gly Leu Leu
65                  70                  75                  80

ggt ttt gcc ttc cat cct gat ttt aaa aat aat cct tat atc tat att     288
Gly Phe Ala Phe His Pro Asp Phe Lys Asn Asn Pro Tyr Ile Tyr Ile
                85                  90                  95

tca ggt aca ttt aaa aat ccg aaa tct aca gat aaa gaa tta ccg aac     336
```

Ser Gly Thr Phe Lys Asn Pro Lys Ser Thr Asp Lys Glu Leu Pro Asn
100 105 110

caa acg att att cgt cgt tat acc tat aat aaa tca aca gat acg ctc 384
Gln Thr Ile Ile Arg Arg Tyr Thr Tyr Asn Lys Ser Thr Asp Thr Leu
115 120 125

gag aag cca gtc gat tta tta gca gga tta cct tca tca aaa gac cat 432
Glu Lys Pro Val Asp Leu Leu Ala Gly Leu Pro Ser Ser Lys Asp His
130 135 140

cag tca ggt cgt ctt gtc att ggg cca gat caa aag att tat tat acg 480
Gln Ser Gly Arg Leu Val Ile Gly Pro Asp Gln Lys Ile Tyr Tyr Thr
145 150 155 160

att ggt gac caa ggg cgt aac cag ctt gct tat ttg ttc ttg cca aat 528
Ile Gly Asp Gln Gly Arg Asn Gln Leu Ala Tyr Leu Phe Leu Pro Asn
165 170 175

caa gca caa cat acg cca act caa caa gaa ctg aat ggt aaa gac tat 576
Gln Ala Gln His Thr Pro Thr Gln Gln Glu Leu Asn Gly Lys Asp Tyr
180 185 190

cac acc tat atg ggt aaa gta cta cgc tta aat ctt gat gga agt att 624
His Thr Tyr Met Gly Lys Val Leu Arg Leu Asn Leu Asp Gly Ser Ile
195 200 205

cca aag gat aat cca agt ttt aac ggg gtg gtt agc cat att tat aca 672
Pro Lys Asp Asn Pro Ser Phe Asn Gly Val Val Ser His Ile Tyr Thr
210 215 220

ctt gga cat cgt aat ccg cag ggc tta gca ttc act cca aat ggt aaa 720
Leu Gly His Arg Asn Pro Gln Gly Leu Ala Phe Thr Pro Asn Gly Lys
225 230 235 240

tta ttg cag tct gaa caa ggc cca aac tct gac gat gaa att aac ctc 768
Leu Leu Gln Ser Glu Gln Gly Pro Asn Ser Asp Asp Glu Ile Asn Leu
245 250 255

att gtc aaa ggt ggc aat tat ggt tgg ccg aat gta gca ggt tat aaa 816
Ile Val Lys Gly Gly Asn Tyr Gly Trp Pro Asn Val Ala Gly Tyr Lys
260 265 270

gat gat agt ggc tat gct tat gca aat tat tca gca gca gcc aat aag 864
Asp Asp Ser Gly Tyr Ala Tyr Ala Asn Tyr Ser Ala Ala Ala Asn Lys
275 280 285

tca att aag gat tta gct caa aat gga gta aaa gta gcc gca ggg gtc 912
Ser Ile Lys Asp Leu Ala Gln Asn Gly Val Lys Val Ala Ala Gly Val
290 295 300

cct gtg acg aaa gaa tct gaa tgg act ggt aaa aac ttt gtc cca cca 960
Pro Val Thr Lys Glu Ser Glu Trp Thr Gly Lys Asn Phe Val Pro Pro
305 310 315 320

tta aaa act tta tat acc gtt caa gat acc tac aac tat aac gat cca 1008
Leu Lys Thr Leu Tyr Thr Val Gln Asp Thr Tyr Asn Tyr Asn Asp Pro
325 330 335

act tgt gga gag atg acc tac att tgc tgg cca aca gtt gca ccg tca 1056
Thr Cys Gly Glu Met Thr Tyr Ile Cys Trp Pro Thr Val Ala Pro Ser
340 345 350

tct gcc tat gtc tat aag ggc ggt aaa aaa gca att act ggt tgg gaa 1104
Ser Ala Tyr Val Tyr Lys Gly Gly Lys Lys Ala Ile Thr Gly Trp Glu
355 360 365

aat aca tta ttg gtt cca tct tta aaa cgt ggt gtc att ttc cgt att 1152

```
Asn Thr Leu Leu Val Pro Ser Leu Lys Arg Gly Val Ile Phe Arg Ile
    370                 375                 380

aag tta gat cca act tat agc act act tat gat gac gct gta ccg atg      1200
Lys Leu Asp Pro Thr Tyr Ser Thr Thr Tyr Asp Asp Ala Val Pro Met
385                 390                 395                 400

ttt aag agc aac aac cgt tat cgt gat gtg att gca agt cca gat ggg      1248
Phe Lys Ser Asn Asn Arg Tyr Arg Asp Val Ile Ala Ser Pro Asp Gly
                405                 410                 415

aat gtc tta tat gta tta act gat act gcc gga gaa gtc caa aaa gat      1296
Asn Val Leu Tyr Val Leu Thr Asp Thr Ala Gly Glu Val Gln Lys Asp
            420                 425                 430

gat ggc tca gta aca aat aca tta gaa aac cca gga tct ctc att aag      1344
Asp Gly Ser Val Thr Asn Thr Leu Glu Asn Pro Gly Ser Leu Ile Lys
        435                 440                 445

ttc acc tat aag gct aag taa tac agt cgc att aaa aaa ccg atc          1389
Phe Thr Tyr Lys Ala Lys     Tyr Ser Arg Ile Lys Lys Pro Ile
    450                     455                 460
```

<210> 14
<211> 454
<212> PRT
<213> Acinetobacter calcoaceticus

<400> 14

```
Asp Val Pro Leu Thr Pro Ser Gln Phe Ala Lys Ala Lys Ser Glu Asn
1               5                   10                  15

Phe Asp Lys Lys Val Ile Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Ile
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ser Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
```

145 150 155 160

Ile Gly Asp Gln Gly Arg Asn Gln Leu Ala Tyr Leu Phe Leu Pro Asn
165 170 175

Gln Ala Gln His Thr Pro Thr Gln Gln Glu Leu Asn Gly Lys Asp Tyr
180 185 190

His Thr Tyr Met Gly Lys Val Leu Arg Leu Asn Leu Asp Gly Ser Ile
195 200 205

Pro Lys Asp Asn Pro Ser Phe Asn Gly Val Val Ser His Ile Tyr Thr
210 215 220

Leu Gly His Arg Asn Pro Gln Gly Leu Ala Phe Thr Pro Asn Gly Lys
225 230 235 240

Leu Leu Gln Ser Glu Gln Gly Pro Asn Ser Asp Asp Glu Ile Asn Leu
245 250 255

Ile Val Lys Gly Gly Asn Tyr Gly Trp Pro Asn Val Ala Gly Tyr Lys
260 265 270

Asp Asp Ser Gly Tyr Ala Tyr Ala Asn Tyr Ser Ala Ala Ala Asn Lys
275 280 285

Ser Ile Lys Asp Leu Ala Gln Asn Gly Val Lys Val Ala Ala Gly Val
290 295 300

Pro Val Thr Lys Glu Ser Glu Trp Thr Gly Lys Asn Phe Val Pro Pro
305 310 315 320

Leu Lys Thr Leu Tyr Thr Val Gln Asp Thr Tyr Asn Tyr Asn Asp Pro
325 330 335

Thr Cys Gly Glu Met Thr Tyr Ile Cys Trp Pro Thr Val Ala Pro Ser
340 345 350

Ser Ala Tyr Val Tyr Lys Gly Gly Lys Lys Ala Ile Thr Gly Trp Glu
355 360 365

Asn Thr Leu Leu Val Pro Ser Leu Lys Arg Gly Val Ile Phe Arg Ile
370 375 380

Lys Leu Asp Pro Thr Tyr Ser Thr Thr Tyr Asp Asp Ala Val Pro Met
385 390 395 400

Phe Lys Ser Asn Asn Arg Tyr Arg Asp Val Ile Ala Ser Pro Asp Gly
405 410 415

Asn Val Leu Tyr Val Leu Thr Asp Thr Ala Gly Glu Val Gln Lys Asp

420 425 430

Asp Gly Ser Val Thr Asn Thr Leu Glu Asn Pro Gly Ser Leu Ile Lys
435 440 445

Phe Thr Tyr Lys Ala Lys
450

<210> 15
<211> 27
<212> DNA
<213> Acinetobacter calcoaceticus

<400> 15
gatactgccg gagctgtcca aaaagat 27

<210> 16
<211> 27
<212> DNA
<213> Acinetobacter calcoaceticus

<400> 16
atctttttgg acagctccgg cagtatc 27

<210> 17
<211> 27
<212> DNA
<213> Acinetobacter calcoaceticus

<400> 17
gatactgccg gaaaggtcca aaaagat 27

<210> 18
<211> 27
<212> DNA
<213> Acinetobacter calcoaceticus

<400> 18
atctttttgg acctttccgg cagtatc 27

<210> 19
<211> 27
<212> DNA
<213> Acinetobacter calcoaceticus

<400> 19
gatactgccg gatatgtcca aaaagat 27

<210> 20
<211> 27
<212> DNA
<213> Acinetobacter calcoaceticus

<400> 20
atctttttgg acatatccgg cagtatc 27

<210> 21
<211> 27
<212> DNA
<213> Acinetobacter calcoaceticus

<400> 21
gatactgccg gagacgtcca aaaagat 27

<210> 22
<211> 27
<212> DNA
<213> Acinetobacter calcoaceticus

<400> 22
atctttttgg acgtctccgg cagtatc 27

<210> 23
<211> 27
<212> DNA
<213> Acinetobacter calcoaceticus

<400> 23
gatactgccg gagaagtcca aaaagat 27

<210> 24
<211> 27
<212> DNA
<213> Acinetobacter calcoaceticus

<400> 24
atctttttgg acttctccgg cagtatc 27

<210> 25
<211> 27
<212> DNA
<213> Acinetobacter calcoaceticus

<400> 25
gatactgccg gatgtgtcca aaaagat 27

<210> 26
<211> 27
<212> DNA
<213> Acinetobacter calcoaceticus

<400> 26
atctttttgg acacatccgg cagtatc 27

## Description

La présente invention se rapporte au domaine de la mise au point d'électrodes à glucose qui présentent un intérêt dans le dosage du glucose, en particulier du glucose sanguin de sujets diabétiques, et pour la mise en oeuvre de biopiles utilisant le glucose comme combustible.

La présente invention vise plus particulièrement des mutants de l'enzyme pyrroloquinoline quinone glucose déshydrogénase soluble (aussi appelée PQQ s-GDH) qui présentent des propriétés avantageuses par rapport à l'enzyme sauvage.

Le diabète de type 2 touche près de deux millions de personnes en France, auxquels s'ajoutent 600 000 qui ignorent leur maladie. Aux Etats-Unis, la situation est encore plus critique. Dans les pays développés, le diabète est la première cause de cécité chez les 20-65 ans.

Le suivi et la surveillance de la maladie repose, entre autres, sur le dosage quotidien du glucose sanguin et l'injection d'insuline. Différentes compagnies proposent des capteurs à glucose permettant aux patients de mesurer chez eux leur glycémie. Ces capteurs peuvent être ampérométriques, potentiométriques ou coulométriques ; ils reposent tous sur l'utilisation d'une enzyme capable d'oxyder le glucose ; les deux principales enzymes étant la glucose oxydase et la PQQ s-GDH.

Les pyrroloquinoline quinone glucose déshydrogénases appartiennent à la famille des quinoprotéines qui ont été identifiées chez *Acinetobacter calcoaceticus ;* il en existe deux types, l'un est une enzyme membranaire et l'autre est une enzyme soluble. Elles catalysent l'oxydation du glucose en D-gluconolactone et peuvent être utilisées comme fournisseur d'électrons.

La PQQ s-GDH d'*Acinetobacter calcoaceticus* est composée d'un homodimère constitué de deux sous-unités d'environ 50 kDa (apoenzyme) et d'un cofacteur pyrroloquinoline quinone (PQQ) (Oubrié et al., J. Mol. Biol; 289, 319-333 (1999)). Cette enzyme comporte un site actif au sein duquel l'oxydation du glucose est catalysée en présence de PQQ ; ce site actif est composé des acides aminés situés aux positions 76, 144, 169, 343, 346 et 428.

La PQQ s-GDH présente un grand intérêt au niveau industriel car elle est facile à obtenir en grande quantité ; elle est ainsi devenue la principale enzyme utilisée dans les capteurs à glucose pour l'auto-surveillance glycémique. L'avantage des quinoprotéines est leur indépendance vis-à-vis de l'oxygène, contrairement à la glucose oxydase qui utilise l'$O_2$ comme accepteur d'électrons.

Sur la base de ses propriétés physico-chimiques, il est également envisageable d'utiliser la PQQ s-GDH pour la préparation de pile à biocombustibles dont le principe repose sur l'oxydation du glucose pour produire un courant électrique.

L'inconvénient de la PQQ s-GDH est sa faible stabilité en température, sa faible stabilité aux pH physiologiques et son activité modérée.

Plusieurs auteurs ont cherché à mettre au point des variants de la PQQ s-GDH afin d'améliorer ses propriétés.

Par mutant ou variant, on entend une PQQ s-GDH dont la séquence protéique comprend l'insertion, la délétion et/ou la substitution d'au moins un acide aminé par rapport à la séquence protéique de la PQQ s-GDH sauvage ; par la suite, les séquences nucléotidique et protéique de référence de la PQQ s-GDH sont celles de la PQQ s-GDH sauvage d'*Acinetobacter calcoaceticus* (respectivement SEQ. ID. N°1 et 2).

- <u>Mutations visant à améliorer la stabilité de la PQQ s-GDH</u>

Le Brevet US 7,244,600 décrit une PQQ s-GDH mutée de telle sorte que les deux sous-unités sont reliées entre elles par un pont disulfure. La mutation consiste à remplacer au moins un acide aminé aux positions 415 et 414 et/ou simultanément les deux acides aminés situés aux positions 340 et 418 par un résidu cystéine. Ces modifications confèrent à l'enzyme une meilleure stabilité thermique.

- <u>Mutation visant à rendre la PQQ s-GDH moins sensible à l'inhibition par son substrat</u>

Le Brevet US 7,244,581 décrit une PQQ s-GDH dans laquelle au moins un acide aminé de la région 349-377 est remplacé par un acide aminé différent. Cette modification conduit à une enzyme qui est moins sensible à l'inhibition par le substrat et peut ainsi être utilisée en présence de fortes concentrations en glucose.

- <u>Mutations visant à rendre la PQQ s-GDH plus spécifique à son substrat naturel (le glucose)</u>

Il est également possible de modifier la PQQ s-GDH sauvage pour la rendre plus spécifique au substrat. Une enzyme spécifique à un substrat ne catalysera que la réaction impliquant ledit substrat, a contrario, une enzyme peu spécifique au substrat est susceptible de catalyser des réactions à partir de substrats structurellement proches du substrat naturel. Le substrat naturel de la PQQ s-GDH est le glucose mais la PQQ s-GDH sauvage est peu spécifique et est également susceptible de catalyser l'oxydation d'autres monosaccharides et de disaccharides.

Plusieurs auteurs ont proposé de modifier la PQQ s-GDH sauvage afin de la rendre plus spécifique au glucose :

- ainsi, la Demande de Brevet US 2007/0105173 propose de modifier la PQQ s-GDH en substituant au moins un des acides aminés situés aux positions 49, 67 à 69, 76, 89, 129 à 131, 167 à 170, 174, 188, 189, 207, 215, 245, 249, 300, 341 à 343, 349, 351 et 429 et/ou en introduisant un acide aminé entre les positions 428 et 429 ;
- la Demande de Brevet EP 1 367 120 propose de modifier la PQQ s-GDH en insérant une leucine, une lysine ou une alanine entre les positions 428 et 429 ;
- le Brevet US 7,037,698 décrit une substitution de l'acide aminé à l'une des positions 75, 326 à 354, 278 à 320 et 162 à 197 de la PQQ s-GDH ;
- la Demande de Brevet US 2007/0243566 recommande d'insérer un acide aminé entre les positions 428 et 429 et, optionnellement, de substituer l'acide aminé en position 428 par une leucine, proline ou valine de la PQQ s-GDH ;
- la Demande Internationale WO 2006/085509 décrit des mutants de la PQQ s-GDH d'*Acinetobacter* comportant une ou plusieurs substitutions aux positions 125, 128, 142, 168, 169, 170, 224, 230, 236, 345, 351, 416 ou 428 ; ce document liste ensuite des mutants spécifiques comportant de nombreux sites mutés ;
- la Demande de Brevet EP 1 666 586 cite des mutants de la PQQ s-GDH d'*Acinetobacter baumanii* portant l'insertion d'une leucine, d'une alanine ou d'une lysine entre les positions 428 et 429 ou encore des mutants pour lesquels l'acide aminé de la position 429 est substituée par une phénylalanine, une proline, une leucine ou une tyrosine ;
- le Brevet US 7,132,270 propose de substituer les acides aminés de la position 348 par une alanine, une glycine ou une sérine et de la position 428 par une leucine, une proline ou une valine.

Pour leur utilisation dans des capteurs à glucose, il est nécessaire de disposer de mutants PQQ s-GDH plus actifs, c'est-à-dire qui permettent une réaction de transformation de glucose en D-gluconolactone plus rapide ce qui n'est pas possible avec les mutants existants.

Il demeure ainsi nécessaire de mettre au point une PQQ s-GDH qui présenterait une meilleure activité que la PQQ s-GDH sauvage tout en gardant une stabilité à la température et au pH satisfaisante voire améliorée.

Pour répondre à ce besoin, les Inventeurs ont mis au point des nouveaux mutants de la PQQ s-GDH sauvage d'*Acinetobacter calcoaceticus ;* ces mutants sont tels que l'asparagine située en position 428 est substituée par une cystéine, une tyrosine, une alanine, un aspartate ou un glutamate.

Ainsi, un premier objet de l'invention se rapporte à un mutant PQQ s-GDH caractérisé en ce que son acide aminé situé en position 428, en référence à la séquence protéique de la PQQ s-GDH sauvage d'*Acinetobacter calcoaceticus* (SEQ. ID. N°2), est substitué par un acide aminé sélectionné dans le groupe constitué par une cystéine, une tyrosine, une alanine, un aspartate ou un glutamate.

Dans le cadre de l'étude ayant conduit à la présente invention, les Inventeurs ont également préparé un mutant PQQ s-GDH caractérisé en ce que son acide aminé situé en position 428, en référence à la séquence protéique de la PQQ s-GDH sauvage d'*Acinetobacter calcoaceticus* (SEQ. ID. N°2), est substitué par une lysine (voir les exemples, partie 4) ; ce mutant a pour séquence protéique la SEQ. ID. N°10 et est codé par la molécule d'acide nucléique de SEQ. ID. N°9. Ce mutant peut être préparé avec les oligonucléotides de SEQ. ID. N°17 et 18 présentés dans le Tableau I ci-après.

La numérotation des acides aminés se réfère à la séquence de la PQQ s-GDH sauvage d'*Acinetobacter calcoaceticus*.

Ainsi, l'invention se rapporte à un mutant PQQ s-GDH ayant une séquence en acides aminés choisie parmi les SEQ. ID. N° 4, 6, 8, 12 et 14, correspondants respectivement aux séquences en acides aminés des mutants cystéine, tyrosine, alanine, aspartate et glutamate de la PQQ s-GDH ; ces enzymes mutées sont codées par des fragments nucléotidiques obtenus par mutation du gène de la PQQ s-GDH d'*Acinetobacter calcoaceticus* avec des paires d'oligonucléotides adaptées.

Ces nouveaux mutants PQQ s-GDH selon l'invention présentent des performances améliorées par rapport à l'enzyme sauvage d'*Acinetobacter calcoaceticus* qui est l'enzyme utilisée dans les capteurs de glucose du commerce :

- leur activité est supérieure à celle de l'enzyme sauvage, en particulier, à des concentrations physiologiques en glucose, soit entre 1 et 10 mM ;

L'activité de l'enzyme peut être quantifiée en suivant la coloration de réactifs d'oxydoréduction survenant au cours de la réaction d'oxydation du glucose en gluconolactone par la PQQ s-GDH ; les réactifs d'oxydoréduction sont par exemple le phénazine méthosulfate (PMS) en combinaison avec le 2,6-dichlorophénolindophénol (DCIP), le ferricyanure de potassium, et le ferrocène.

- ils sont moins sensibles à l'effet inhibiteur du glucose à concentration élevée.

L'évaluation de la spécificité d'un mutant PQQ s-GDH pour un substrat peut être réalisée en comparant l'activité enzymatique dudit mutant sur le glucose et sur plusieurs autres sucres tels que des disaccharides en tant que substrat.

Les propriétés avantageuses des mutants PQQ s-GDH selon l'invention rendent leur utilisation particulièrement adaptée à des systèmes bioélectriques tels que des biopiles utilisant le glucose comme source d'énergie et des biocapteurs de glucose.

La présente invention se rapporte également à une molécule d'acide nucléique codant pour un mutant PQQ s-GDH selon l'invention ; ladite molécule d'acide nucléique étant obtenue par modification d'une PQQ s-GDH sauvage, telle que celle d'*Acinetobacter calcoaceticus*, avec une paire d'oligonucléotides sélectionnée dans le groupe constitué des paires d'oligonucléotides représentées dans le Tableau I.

Tableau I: liste et séquence des oligonucléotides utilisés pour la préparation des mutants PQQ s-GDH selon l'invention.

| Oligonucléotides | séquences |
|---|---|
| Paire d'oligonucléotides utilisés pour la préparation du mutant alanine (N428A) | |
| N428A Sens | gAT ACT gCC ggA gCT gTC CAA AAA gAT (SEQ. ID. N°15) |
| N428A Antisens | ATC TTT TTg gAC AgC TCC ggC AgT ATC (SEQ. ID. N°16) |
| Paire d'oligonucléotides utilisés pour la préparation du mutant lysine (N428K) | |
| N428K Sens | gAT ACT gCC ggA AAG gTC CAA AAA gAT (SEQ. ID. N°17) |
| N428K Antisens | ATC TTT TTg gAC CTT TCC ggC AgT ATC (SEQ. ID. N°18) |
| Paire d'oligonucléotides utilisés pour la préparation du mutant tyrosine (N428Y) | |
| N428Y Sens | gAT ACT gCC ggA TAT gTC CAA AAA gAT (SEQ. ID. N°19) |
| N428Y Antisens | ATC TTT TTg gAC ATA TCC ggC AgT ATC (SEQ. ID. N°20) |
| Paire d'oligonucléotides utilisés pour la préparation du mutant aspartate (N428D) | |
| N428D Sens | gAT ACT gCC ggA gAC gTC CAA AAA gAT (SEQ. ID. N°21) |
| N428D Antisens | ATC TTT TTg gAC gTC TCC ggC AgT ATC (SEQ. ID. N°22) |
| Paire d'oligonucléotides utilisés pour la préparation du mutant glutamate (N428E) | |
| N428E Sens | gAT ACT gCC ggA gAA gTC CAA AAA gAT (SEQ. ID. N°23) |
| N428E Antisens | ATC TTT TTg gAC TTC TCC ggC AgT ATC (SEQ. ID. N°24) |
| Paire d'oligonucléotides utilisés pour la préparation du mutant cystéine (N428C) | |
| N428C Sens | gAT ACT gCC ggA TgT gTC CAA AAA gAT (SEQ. ID. N°25) |
| N428C Antisens | ATC TTT TTg gAC ACA TCC ggC AgT ATC (SEQ. ID. N°26) |

Les molécules d'acide nucléique codant pour les mutants PQQ s-GDH selon l'invention peuvent être notamment préparées en modifiant la séquence nucléotidique du gène codant pour l'enzyme sauvage de séquence SEQ. ID. N°1 produite par *Acinetobacter calcoaceticus.* Plusieurs techniques permettant la modification de la séquence du gène sont connues de l'homme du métier (voir la revue de Igarashi et al., Archives of Biochemistry and Biophysocs 428 (2004) 52-63). Selon un mode particulier de préparation, les molécules d'acide nucléique codant pour les mutants PQQ s-GDH selon l'invention sont préparées par mutagénèse par PCR en présence d'un oligonucléotide portant la mutation à introduire (voir l'exemple ci-après).

Selon un mode de réalisation particulier, la présente invention se rapporte à une molécule d'acide nucléique codant pour un mutant PQQ s-GDH selon l'invention dont la séquence est sélectionnée dans le groupe constitué des séquences SEQ. ID. N°3, 5, 7, 11 et 13. Les molécules d'acide nucléique codant pour les mutants PQQ s-GDH selon l'invention peuvent alors être clonées dans un vecteur d'expression tel qu'un plasmide, puis transformé dans un hôte approprié tel qu'une bactérie, une levure ou encore une culture cellulaire.

Par vecteur d'expression, on entend un vecteur possédant une région permettant l'insertion d'une séquence nucléotidique codante entre les signaux indispensables à son expression, notamment, un promoteur (constitutif ou inductible), un site de fixation des ribosomes, un signal de terminaison de transcription et, éventuellement, un marqueur de sélection tel qu'un gène de résistance à un antibiotique.

La présente invention se rapporte encore à un vecteur d'expression comprenant ladite molécule d'acide nucléique et à une cellule hôte transformée avec ledit vecteur d'expression et exprimant un mutant PQQ s-GDH selon l'invention.

L'introduction du vecteur d'expression dans la cellule hôte peut être réalisée par toute méthode connue de l'homme du métier, en particulier, par modification de la perméabilité membranaire de la cellule hôte, par exemple en présence d'ions calcium, ou par électroporation.

Après culture des cellules hôtes transformées pour exprimer un mutant PQQ s-GDH selon l'invention, lesdites cellules peuvent être récupérées par centrifugation, lysées afin de libérer les enzymes dont ledit mutant PQQ s-GDH selon l'invention.

Si *Escherichia coli* est le micro-organisme hôte, les plasmides qui peuvent être utilisés sont notamment les plasmides pBluescript, pUC18 ou similaires.

A titre d'exemple, les cellules hôtes qui peuvent être utilisées comprennent *Escherichia coli* W3110, *Escherichia coli* C600, *Escherichia coli* JM109, *Escherichia coli* JM101, *Escherichia coli* DH5$\alpha$...

De préférence, les mutants PQQ s-GDH selon l'invention sont produits dans une souche d'*Escherichia coli* JM101 ; la molécule d'acide nucléique qui les code est obtenue par modification du gène de la PQQ s-GDH d'*Acinetobacter calcoaceticus* et clonée dans le vecteur pUC18 (Cleton-Jansen et al., Mol. Gen. Genet. 217 (1989) 430-436). Les mutants ainsi produits sont exportés dans le périplasme de la bactérie grâce à la séquence signal de la PQQ s-GDH. Les mutants sont ensuite purifiés après rupture des bactéries par sonication.

L'invention se rapporte également à l'utilisation d'un mutant PQQ s-GDH selon l'invention pour doser le glucose en solution, c'est-à-dire mesurer la concentration en glucose dans un échantillon, notamment un échantillon biologique, en particulier, dans du sang.

Le dosage en solution du glucose dans un échantillon biologique donné peut être réalisé en introduisant dans ledit échantillon un réactif d'oxydoréduction et un mutant PQQ s-GDH selon l'invention puis en comparant l'intensité de la coloration obtenue avec des solutions standards ayant une teneur en glucose connue.

La présente invention se rapporte également à un kit de dosage en solution du glucose caractérisé en ce qu'il comprend un mutant PQQ s-GDH selon l'invention.

Typiquement, ledit kit de dosage contient en outre les réactifs nécessaires à la mise en oeuvre du test de dosage du glucose, en particulier, les tampons ; tout tampon peut être utilisé dans le kit selon l'invention, on peut citer sans caractère limitatif les tampons phosphates, actétates, tampon au trishydroxyméthylaminométhane (TRIS), à l'acide N-morpholino-3-propane sulfonique (MPOS), à l'acide 4-(2-hydroxyéthyl)-1-pipérazine éthane sulfonique (HEPES), tampon comprenant un mélange de tampons tel que TRIS-acétate..., les réactifs d'oxydoréduction peuvent être tout réactif permettant d'oxyder le mutant PQQ s-GDH, ils peuvent être sélectionnés dans le groupe constitué par phénazine méthosulfate (PMS) en combinaison avec le 2,6-dichlorophénolindophénol (DCIP) ; du ferricyanure de potassium ; du ferrocène et des complexes dérivés du ferrocène tels que ferrocèneméthanol, ferrocène carboxylique acide ; et des complexes d'osmium et de ruthénium, les solutions standard de glucose permettant la réalisation de courbes d'étalonnage, et les instructions d'utilisation nécessaires pour effectuer le dosage.

La présente invention se rapporte encore à des électrodes à glucose comprenant un matériau conducteur tel qu'un métal conducteur, notamment, du platine, du cuivre, de l'argent, de l'aluminium, de l'or ou de l'acier ou du carbone, comme du carbone vitreux, des fibres de carbone, des fibres de nanotubes de carbone ou encore en diamant... ledit matériau conducteur est recouvert d'un dépôt comprenant au moins un mutant PQQ s-GDH selon l'invention ; ledit dépôt pouvant en outre comprendre un polymère redox pour améliorer les propriétés conductrices du matériau conducteur.

Le polymère redox est choisi parmi les polymères à base de ferrocène, d'osmium et de ruthénium et les polymères conducteurs tels que le polypyrrole et la polyananilline.

Les méthodes d'immobilisation du mutant PQQ s-GDH sur ledit matériau conducteur peuvent être choisies parmi les méthodes classiques à la disposition de l'homme du métier qui comprennent notamment l'inclusion du mutant PQQ s-GDH dans une matrice polymérique, l'adsorption du mutant PQQ s-GDH à la surface de la membrane polymérique, la fixation par liaison covalente ou encore l'électrodéposition (Gao *et al.,* Chem. Int. ED. 2002, 41, N°5, 810-813).

De préférence, le mutant PQQ s-GDH immobilisé est constitué de l'apoenzyme assemblée au cofacteur PQQ ;

mais il est également possible d'immobiliser l'apoenzyme seule et de fournir par ailleurs, par exemple en solution dans le milieu réactionnel, le cofacteur PQQ.

De telles électrodes sont avantageusement utilisées dans des systèmes bioélectriques tels que des biopiles à glucose ou des biocapteurs de glucose.

La présente invention se rapporte ainsi également à un biocapteur de glucose comprenant une électrode selon l'invention.

Un biocapteur de glucose est constitué d'une électrode sur laquelle est immobilisé un biorécepteur capable de reconnaître une cible biologique ; la fixation de la cible biologique sur le biorécepteur conduit à des modifications physico-chimiques de la membrane et la production d'un signal électrique par un transducteur électrochimique (ampérométrique, potentiométrique, conductimétrique,...) accolé à l'électrode ; dans le cas présent le biorécepteur est un mutant PQQ s-GDH selon l'invention et la cible biologique est son substrat : le glucose.

Selon une variante de réalisation, l'électrode sur laquelle est immobilisée le mutant PQQ s-GDH est également recouverte d'une membrane qui évite le détachement dudit mutant de l'électrode. Ladite membrane peut être constituée de nafion, de cellulose ou de tout matériau biocompatible, c'est-à-dire compatible avec un environnement physiologique.

Selon une variante de l'invention, le biocapteur de glucose est implanté sous la peau et permet d'enregistrer la concentration en glucose du sang.

La présente invention se rapporte également à des biopiles utilisant du glucose comme source d'énergie et comprenant une première électrode selon l'invention à titre d'anode et une seconde électrode à titre de cathode. La cathode peut être, par exemple, une électrode enzymatique qui permet de réduire l'oxygène portant une enzyme choisie dans la classe des enzymes à base de cuivre (multi coppers oxydases) et particulièrement la bilirubine oxydase et la laccase. Il peut également s'agir d'une électrode métallique, par exemple en platine, en or ou en un alliage de platine ou d'or.

L'invention se rapporte encore à un procédé de dosage en solution du glucose dans un échantillon comprenant les étapes suivantes :

a) introduction dans ledit échantillon d'un réactif d'oxydoréduction dont la réduction conduit à un changement de couleur et d'un mutant PQQ s-GDH selon l'invention ;
b) mesure de l'intensité de la coloration de l'échantillon après réaction enzymatique ;
c) comparaison de l'intensité de coloration mesurée à l'étape b) avec l'intensité mesurée pour des solutions standards ayant une teneur en glucose connue ;
d) détermination de la concentration en glucose dudit échantillon.

Le réactif d'oxydoréduction dont la réduction conduit à un changement de couleur est choisi parmi le phénazine méthosulfate (PMS) en combinaison avec le 2,6-dichlorophénolindophénol (DCIP), le ferricyanure de potassium, et le ferrocène.

L'invention se rapporte également à un procédé de dosage du glucose d'un échantillon, caractérisé en ce qu'il comprend les étapes suivantes :

a) introduction dans ledit échantillon d'une électrode à glucose selon l'invention ;
b) mesure de l'intensité du courant dans l'échantillon ;
c) comparaison de l'intensité du courant mesurée à l'étape b) avec l'intensité mesurée pour des solutions standards ayant une teneur en glucose connue ;
d) détermination de la concentration en glucose dudit échantillon.

Outre les dispositions qui précèdent, l'invention comprend encore d'autres dispositions qui ressortiront de la description qui va suivre, qui se réfèrent à des exemples de mise en oeuvre de la présente invention, ainsi qu'aux figures annexées dans lesquelles :

<u>Figures</u>

Les **Figures 1A et 1B** sont des schémas représentant le site actif de la PQQ s-GDH d'*Acinetobacter calcoaceticus* sauvage **(Figure 1A**) ou mutée en position Asn428Cys **(Figure 1B).** La figure a été créée avec le logiciel PyMol (édité par DeLano Scientific LLC, version de 2006) d'après les coordonnées pdb 1cq1 décrites par Oubrie et *al.* (1999). « G » désigne le glucose et « PQQ », le coenzyme PQQ. La numérotation des résidus acides aminés du site actif correspond à la SEQ. ID. N°2.

Les **Figures 2A** à **2F** sont des graphes représentant les paramètres cinétiques à l'état stationnaire en présence de glucose ou de maltose :

- de la PQQ s-GDH d'*Acinetobacter calcoaceticus* sauvage (WT) et mutée en position 428 par une cystéine (N428C, **Figure 2A) ;**
- de la PQQ s-GDH d'*Acinetobacter calcoaceticus* mutée en position 428 par une tyrosine (N428Y, **Figure 2B) ;**
- de la PQQ s-GDH d'*Acinetobacter calcoaceticus* mutée en position 428 par une alanine (N428A, **Figure 2C) ;**
- de la PQQ s-GDH d'*Acinetobacter calcoaceticus* mutée en position 428 par une lysine (N428K, **Figure 2D)** ;
- de la PQQ s-GDH d'*Acinetobacter calcoaceticus* mutée en position 428 par un aspartate (N428D, **Figure 2E) ;**
- de la PQQ s-GDH d'*Acinetobacter calcoaceticus* mutée en position 428 par un glutamate (N428E, **Figure 2F).**

Les **Figures 3A et 3B** représentent l'activité en fonction du pH de la PQQ s-GDH d'*Acinetobacter calcoaceticus* sauvage (WT) et mutée en position 428 par une cystéine (N428C).
La **Figure 4** représente la courbe d'activité de la PQQ s-GDH sauvage (WT) et mutée par une cystéine en position 428 (N428C) en fonction de la température.
La **Figure 5** est un graphe représentant l'activité de PQQ s-GDH mutée en position 428 par une cystéine (N428C) en fonction de différents substrats.

## EXEMPLES

### 1. Matériels

#### 1.1. Souches bactériennes d'*Escherichia coli*

**DH$_{5\alpha}$** : *supE44,* $\Delta$*lac*U169, ($\Phi$80 *lac*ZDM15), *hsd*R17, *rec*A1, *end*A1, *gyr*A96, *thi*-1, *rel*A1 (Hanahan, 1983). Cette souche est utilisée pour la préparation de plasmide et la mutagenèse dirigée.
**JM101 :** F'*traD36 proA$^+$B$^+$ lac*I$^q$ $\Delta$(l*acZ)M15*/$\Delta$(*lac-proAB*) *glnV thi*. Cette souche est utilisée pour la production en batch de la PQQ glucose déshydrogénase d'*Acinetobacter calcoaceticus.* Cette souche porte le plasmide F-pro-lacI qui inhibe l'expression du promoteur lac.

#### 1.2. Plasmide

**pgp492 :** don de Nora Goosen, plasmide recombinant obtenu par clonage de la séquence codante du gène de la PQQ glucose déshydrogénase soluble d'*Acinetobacter calcoaceticus (*Cleton-Jansen et al., Mol. Gen. Genet. 217 (1989) 430-436).

#### 1.3. Milieu de culture

Milieu riche LB : Tryptone 10 g/L ; Extrait de levure 5 g/L ; NaCl 5 g/L ; $H_2O$ distillée qsp 1L ; pH non ajusté, autoclavé pendant 50 min à 1 bar.

### 2. Techniques de génie génétique

#### 2.1. Transformation des bactéries supercompétentes

Les bactéries *DH$_{5\alpha}$* supercompétentes sont préparées à l'aide de la méthode d'Inoue (Sambrook and Russell (2001). Molecular Cloning: A Laboratory Manual (3rd ed.). Cold Spring Harbor Laboratory Press).

#### 2.2. Préparation de l'ADN

Un kit de purification d'ADN plasmidique (Quiagen) est utilisé pour les préparations d'ADN en petite et grande quantité.

#### 2.3. Séquençage de l'ADN double brin

L'ADN double brin est séquencé avec le kit de séquençage BigDye Terminator v1.1 ou v3.1. Le réactif contient les 4 ddNTPs avec différents marqueurs fluorescents (BigDyeTerminators), l'AmpliTaq DNA Polymerase, et tous les autres composants nécessaires à la réaction. Les produits d'extension doivent être purifiés avant le passage sur séquenceur ABI 3130xl, pour éliminer les marqueurs non incorporés, sels et autres contaminants.

### 2.4. Mutagenèse dirigée par PCR

Dans le protocole de mutagenèse, la PCR est réalisée avec l'ADN polymérase *Pfu* de *Pyrococcus furiosus,* six fois plus fidèle que la polymérase *Taq* pour répliquer les deux brins du plasmide. Les oligodésoxyribonucléotides listés dans le Tableau I, chacun complémentaire d'un brin du plasmide, vont servir d'amorces pour la synthèse de l'ADN par l'ADN polymérase *Pfu*, ce qui conduit à un plasmide muté aux extrémités non religuées.

Le produit amplifié est ensuite traité avec l'endonucléase *Dpn* I (séquence cible 5'$G^{m6}$ ATC-3'), spécifique de l'ADN parental et permet ainsi de sélectionner l'ADN néosynthétisé contenant la mutation. L'ADN isolé de la plupart des souches d'E. *coli* est « DAM méthylé » (DAM pour DNA adenine methylase ; indique la présence d'un méthyle sur les adénines des séquences GATC) et donc susceptible d'être digéré par *Dpn* I. Une fraction du mélange de digestion est utilisée pour transformer des bactéries $DH_{5\alpha}$ supercompétentes qui vont religuer les extrémités du plasmide muté.

### 3. Production, purification et caractérisation de la PQQ s-GDH d'*Acinetobacter calcoaceticus*

### 3.1. Production des PQQ s-GDH de type sauvage et mutées

L'apoenzyme s-GDH est produite dans la souche *E. coli* JM101 par le plasmide recombinant pgp492 portant la séquence codant le s-GDH sauvage ou mutée. Une préculture de 50 mL de milieu LB supplémenté en ampicilline (200 mg/L) (LBA) est ensemencée par un clone isolé sur boîte LB agar supplémenté en ampicilline (100 mg/mL) et laissée sous agitation, à 220 rpm, sur la nuit à 37°C. Les cultures sont ensemencées au $1/100^{ème}$ en milieu LBA. Elles sont incubées à 37°C sous agitation (220 rpm) jusqu'à une $DO_{600nm}$ comprise entre 0.4 et 1 $DO_{600nm}$ /mL. Les cultures sont ensuite induites par 400 μM d'IPTG puis laissées sous agitation (180-220 rpm) à 25°C pendant 20H. Les cellules récoltées par centrifugation (5285 g, 4°C) sont lavées dans un tampon Tris 20 mM, $CaCl_2$ 3 mM pH 7.5 et stockées à -20°C ou à -80°C sans additif.

### 3.2. Purification des enzymes PQQ s-GDH de type sauvage et mutées

3.2.1. Eclatement des cellules et traitement à la DNase I

Le culot cellulaire, issu d'un litre de culture, est repris dans 20 mL de tampon Tris 20 mM, $CaCl_2$ 3 mM, pH 7.5 et soniqué sous une puissance de sonication de 40 W pendant 3 minutes par période d'une seconde d'ultrasons et d'une seconde d'arrêt. L'échantillon obtenu appelé extrait brut est complémenté par 2 mM final $MgCl_2$ et traité 30 minutes à température ambiante à la DNase I (1 U/mL d'extrait brut). L'extrait brut est ensuite débarrassé des débris cellulaires insolubles par 45 minutes de centrifugation à 20000 g.

3.2.2. Chromatographie échangeuse de cations

Le surnageant de sonication filtré sur filtre ayant un seuil de coupure de 0,22 μm (Millex-GS 0,22 μm, Millipore) et dilué à une $DO_{280nm}$ de 10 est injectée sur une colonne échangeuse de cations source 30S (GE Healthcare®), couplée au système AKTA purifier (GE Healthcare®), équilibrée dans un tampon Tris 20 mM, $CaCl_2$ 3 mM, pH 7.5. L'élution est réalisée par un gradient de NaCl de 0 à 1 M dans le même tampon sous un débit de 5 mL/min. Les fractions contenant la protéine s-GDH sont rassemblées et concentrées par filtration par centrifugation sur membrane Amicon YM10. A ce stade, la protéine s-GDH est pure et peut être conservée à -20°C sous forme précipitée en présence de sulfate d'ammonium (90% de saturation).

3.2.3 Caractérisation des enzymes s-GDH de type sauvage et mutées

3.2.3.1. Détermination de la masse moléculaire

La masse moléculaire du monomère de la s-GDH après purification est déterminée par spectrométrie de masse MALDI. La protéine est dessalée sur colonne PD10 (GE Healthcare) et éluée dans un tampon bicarbonate d'ammonium 50 mM pH 7.5. L'échantillon lyophilisé est ensuite analysé par MALDI-TOF et une masse de 50234,83 Da est trouvée ; cette masse correspond à la masse théorique de la PQQ s-GDH sans le peptide signal.

3.2.3.2. Reconstitution de la s-GDH avec le cofacteur PQQ

En sortie de purification (tampon Tris 20 mM, $CaCl_2$ 3 mM pH 7.5), la solution d'enzyme est préincubée 15 minutes avec du PQQ à température ambiante. Le PQQ ajouté correspond à une concentration finale équivalente à deux fois en molarité la concentration en enzyme. L'excès de PQQ est ensuite éliminé par dessalage sur une colonne PD10 (GE Healthcare) équilibrée dans un tampon Pipes 20 mM, $CaCl_2$ 3 mM pH 7.

3.2.3.3. Mesure de la concentration

La concentration en enzyme d'une solution est calculée à partir de la DO mesurée à 280 nm en utilisant pour l'enzyme (dimérique ou monomérique) avec ou sans cofacteur PQQ un coefficient d'extinction de 1,28 ou 1,67 $l.g^{-1}.cm^{-1}$ respectivement (Olsthoorn et *al,* 1997).

3.2.3.4. Test enzymatique

Les tests enzymatiques sont effectués à l'aide d'un spectrophotomètre Varian dans un tampon phosphate de sodium 20 mM pH 7 à 37°C dans un volume de 3 mL en suivant la disparition par réduction du DCIP par l'intermédiaire du PMS, qui intervient comme accepteur d'électrons, à 600 nm en fonction du temps. L'activité spécifique de l'enzyme est exprimée en μmoles de DCIP disparu par minute et par mg de protéine. Les concentrations en PMS et DCIP sont de 0,6 et de 0,06 mM respectivement. L'enzyme est diluée de façon à mesurer une pente comprise entre - 0,05 et - 0,2 $DO_{600nm}$/min.

**4. Techniques d'étude des propriétés enzymatiques des PQQ s-GDH de type sauvage et mutées**

**4.1. Détermination des constantes cinétiques ($k_{cat}$) et de Michaelis ($K_M$) à l'état stationnaire**

Les expériences sont réalisées à 37°C sur un spectrophotomètre Varian dans un tampon phosphate de sodium 20 mM pH 7. La concentration en substrat (glucose et maltose) dans le test varie entre 0 et 800 mM.

Le PMS est utilisé comme premier accepteur d'électron et l'activité enzymatique est suivie à 600 nm par la réduction du DCIP utilisé comme deuxième accepteur d'électrons. Les concentrations initiales de PMS et de DCIP sont respectivement de 0,6 et de 0,06 mM. Le test est déclenché par l'ajout d'enzyme. Les points expérimentaux sont analysés par régression non linéaire aux équations 1, 2 ou 3 à l'aide du logiciel Sigma-plot 6.0 selon les équations suivantes :

(1) Modèle de Michaelis-Menten

$$k_{ss} = k_{cat} * [S] / (K_M + [S])$$

(2) Double hyperbole

$$k_{ss} = k_{cat1} * [S] / (K_{M1} + [S]) + k_{cat2} * [S] / K_{M2} + [S])$$

(3) Modèle de Michaelis-Menten avec inhibition compétitive

$$v/v_{max} = [S] / K_S * (1+ [S] / K_S + [I] / K_I)$$

*Résultats*

Les graphes des **Figures 2A, 2B, 2C, 2D, 2E et 2F** représentent les paramètres cinétiques à l'état stationnaire de la PQQ glucose déshydrogénase soluble d'*Acinetobacter calcoaceticus* sauvage (WT) et mutée en position 428 (N428C, N428Y, N428A, N428K, N428D et N428E) en présence de glucose ou de maltose.

Ces résultats montrent que, pour les deux substrats, maltose et glucose, les enzymes mutées selon l'invention présentent une activité supérieure à celle de l'enzyme sauvage ; certains de ces mutants sont deux fois plus actifs que l'enzyme sauvage.

### 4.2. Etude en fonction du pH

4.2.1. Activité en fonction du pH

L'étude de la variation de la constante de vitesse de la réaction en fonction du pH est réalisée sur une gamme de pH allant de 5 à 9 par incubation des enzymes sauvages et mutées soit dans un tampon mixte composé de Tris 120 mM, imidazole 30 mM, acide acétique 30 mM (TIA), dont la force ionique est ajustée à 190 mM avec du NaCl, soit dans un tampon sodium phosphate 20 mM allant de pH 6 à 8 (NaPi). Les expériences sont réalisées à 37°C à l'aide d'un spectrophotomètre Varian. Le PMS est utilisé comme premier accepteur d'électrons à une concentration de 0.6 mM. L'activité est suivie par la disparition du DCIP à 600 nm utilisé comme deuxième accepteur d'électrons à une concentration de 0,06 mM. Le test est déclenché par ajout de l'enzyme. L'activité optimale pour l'enzyme sauvage ou mutée correspond à 100% et l'activité relative à chaque pH est représentée.

*Résultats*

Les **Figures 3A et 3B** sont des graphes représentant l'activité en fonction du pH de la PQQ s-GDH d'*Acinetobacter calcoaceticus* sauvage (WT) et mutée en position 428 par une cystéine (N428C). Ces résultats montrent que le mutant cystéine de la PQQ s-GDH (N428C) présente une activité relative comparable à celle de l'enzyme sauvage démontrant que la mutation ne conduit pas à une perte d'activité relative quel que soit le pH.

4.2.2. Stabilité en fonction du pH

La stabilité en fonction du pH de la PQQ s-GDH sauvage ou mutée est déterminée par dilution de l'enzyme purifiée à homogénéité dans un tampon mixte allant de pH 5 à 9. Ce tampon mixte est composé de Tris 120 mM, imidazole 30 mM, acide acétique 30 mM, dont la force ionique est ajustée à 190 mM avec du NaCl. La solution d'enzyme diluée, entre 1 et 6 μg/ml, est préincubée à 37°C. Différents prélèvements sont effectués en fonction du temps. L'activité résiduelle est mesurée à 37°C à l'aide d'un spectrophotomètre Varian dans un tampon phosphate de sodium 20 mM pH 7 en présence de 0,06 mM DCIP, 0,6 mM PMS. La concentration en glucose est de 75 mM pour tester l'enzyme sauvage et de 150 mM pour tester le mutant N428C.

*Résultats*

L'enzyme mutée selon l'invention présente une meilleure stabilité que l'enzyme sauvage quel que soit le pH et que cette stabilité est constante sur la gamme de pH testée, en particulier, l'enzyme mutée reste stable même à des pH supérieurs à 7.

### 4.3. Etude en fonction de la température

4.3.1. Activité en fonction de la température

L'étude de la variation de la constante de vitesse de la réaction en fonction du pH est réalisée dans un tampon phosphate de sodium 20 mM pH 7, en présence de 0,06 mM de DCIP et de 0,6 mM de PMS. La concentration en glucose est de 75 mM pour tester l'enzyme sauvage et de 150 mM pour tester le mutant N428C. La température varie de 10 à 60°C. Le suivi de l'activité s'effectue sur un spectrophotomètre Varian CARY UV Biomelt régulé en température. Le test est déclenché par ajout de l'enzyme.

*Résultats*

La **Figure 4** représente la courbe d'activité de la PQQ s-GDH sauvage (WT) et mutée (N428) en fonction de la température.

Cette courbe montre qu'entre 30 et 50°C, l'enzyme mutée présente une meilleure activité que l'enzyme sauvage.

4.3.2. Stabilité de l'enzyme en fonction de la température

L'étude est réalisée dans un tampon phosphate de sodium 20 mM pH 7, en présence de 0,06 mM de DCIP et de 0,6 mM de PMS. La concentration en glucose est de 75 mM pour tester l'enzyme sauvage et de 150 mM pour tester le mutant N428C. La température varie de 10 à 60°C. Le suivi de l'activité s'effectue à 37°C sur un spectrophotomètre Varian CARY UV Biomelt régulé en température. Le test est déclenché par ajout de l'enzyme.

*Résultats*

On constate que pendant un temps d'incubation inférieur à 200 minutes, l'enzyme mutée N428C présente une meilleure activité à 40 et 50°C que l'enzyme sauvage démontrant ainsi son intérêt pour des kits de dosage du glucose sanguin.

**4.4. Etude en fonction du substrat**

La spécificité du mutant cystéine (N428C) par rapport à différents substrats a été évaluée.
Le protocole mis en oeuvre pour cette mesure de spécificité est identique à celui décrit au point 4.1. en utilisant des substrats différents.
Les résultats obtenus sont représentés dans le Tableau II ci-dessous et à la **Figure 5.**

Tableau **II :** Comparaison de la spécificité de substrat de la PQQ s-GDH d'*Acinetobacter calcoaceticus* sauvage et mutée comprenant une cystéine en position 428 (mutant N428C).

| | **WT** | | **N428C** | | |
|---|---|---|---|---|---|
| **Substrat** | ***k*ss $s^{-1}$ à 4mM substrat** | **%Activité relative / WT Glucose** | ***k*ss $s^{-1}$ à 4 mM substrat** | **% Activité relative / WT** | **% Activité relative / N428C Glucose** |
| **Glucose** | 806 | 100 | 1396 | 173 | 100 |
| **Maltose** | 660 | 81 | 861 | 130 | 61 |
| **Ribose** | 86 | 10 | 67 | 77 | 4 |
| **Xylose** | 148 | 18 | 159 | 107 | 11 |
| **Galactose** | | | 357 | 26 | 25 |

*Résultats*

Même si les valeurs brutes des $k_{ss}$ du mutant N428C pour la plupart des substrats sont plus élevées que celles de l'enzyme sauvage, l'activité relative, qui caractérise la spécificité d'une enzyme pour un substrat par rapport à un autre substrat, montre que le mutant N428C est plus spécifique au glucose. Ainsi, à 4 mM de substrat, avec le maltose comme substrat, on mesure une activité relative par rapport au glucose de 81% pour l'enzyme sauvage alors que le mutant N428C présente une activité relative du maltose par rapport au glucose de 61%.
Ce même protocole a été mis en oeuvre pour évaluer la spécificité au glucose et au maltose des mutants aspartate (N428D), glutamate (N428E), alanine (N428A), Tyrosine (N428Y) et lysine (N428K).
Les résultats sont présentés dans le Tableau III ci-dessous :

| | **N428D** | | | **N428E** | | |
|---|---|---|---|---|---|---|
| **substrat** | kss (s-1) à **5mM** substrat | activité relative / N428D glucose (%) | Activité relative / WT (%) | kss (s-1) à **5mM** substrat | Activité relative / N428E glucose (%) | Activité relative / WT (%) |
| **glucose**<br>**maltose** | 1214<br>1103 | 100<br>91 | 150<br>167 | 1380<br>1182 | 100<br>85 | 171<br>179 |
| | **N428A** | | | **N428Y** | | |
| **substrat** | kss ($s^{-1}$) à **5mM** substrat | Activité relative / N428A glucose (%) | Activité relative / WT (%) | kss ($s^{-1}$) à **5mM** substrat | Activité relative / N428Y glucose (%) | Activité relative / WT (%) |
| **glucose**<br>**maltose** | 1058<br>638 | 100<br>60 | 131<br>97 | 1353<br>716 | 100<br>52 | 167<br>108 |

(suite)

| substrat | N428K | | | |
|---|---|---|---|---|
| | kss ($s^{-1}$) à **5mM** substrat | Activité relative / N428Y glucose (%) | Activité relative / WT (%) | |
| **glucose** | 940 | 100 | 116 | |
| **maltose** | 625 | 66 | 94 | |

## Revendications

1. Mutant de la PQQ s-GDH, **caractérisé en ce que** son acide aminé situé en position 428, en référence à la SEQ. ID. N°2, est substitué et qu'il a une séquence en acides aminés sélectionnée dans le groupe constitué par les SEQ. ID. N°4, 6, 8, 12 et 14.

2. Molécule d'acide nucléique, **caractérisée en ce qu'**elle code pour un mutant PQQ s-GDH selon la revendication 1.

3. Molécule d'acide nucléique selon la revendication 2, **caractérisée en ce qu'**elle est obtenue par mutation de la molécule d'acide nucléique de séquence SEQ. ID. N°1 avec une paire d'oligonucléotides sélectionnée dans le groupe constitué par les paires de SEQ. ID. N° 15 et 16 ; 19 et 20 ; 21 et 22 ; 23 et 24 et 25 et 26.

**4.** Molécule d'acide nucléique selon la revendication 3, **caractérisée en ce qu'**elle a une séquence sélectionnée dans le groupe constitué par les SEQ. ID. N°3, 5, 7, 11 et 13.

**5.** Vecteur d'expression, **caractérisé en ce qu'**il comprend une molécule d'acide nucléique selon l'une quelconque des revendications 2 à 4.

**6.** Cellule hôte exprimant un mutant de la PQQ s-GDH selon la revendication 1, **caractérisée en ce qu'**elle est transformée avec un vecteur d'expression selon la revendication 5.

**7.** Utilisation d'un mutant de la PQQ s-GDH selon la revendication 1 pour mesurer la concentration en glucose dans un échantillon.

**8.** Utilisation selon la revendication 7, **caractérisée en ce que** l'échantillon est biologique, en particulier, est du sang.

**9.** Kit de dosage du glucose **caractérisé en ce qu'**il comprend un mutant de la PQQ s-GDH selon la revendication 1.

**10.** Electrode à glucose, **caractérisée en ce qu'**elle comprend un matériau conducteur recouvert d'un dépôt comprenant au moins un mutant de la PQQ s-GDH selon la revendication 1.

**11.** Capteur de glucose, **caractérisé en ce qu'**il est constitué d'une électrode selon la revendication 10.

**12.** Biopile à glucose, **caractérisée en ce qu'**elle comprend une première électrode selon la revendication 10 à titre d'anode et une seconde électrode à titre de cathode.

**13.** Procédé de dosage en solution du glucose d'un échantillon, **caractérisé en ce qu'**il comprend les étapes suivantes :

a) introduction dans ledit échantillon d'un réactif d'oxydoréduction dont la réduction conduit à un changement de couleur et d'un mutant de la PQQ s-GDH selon la revendication 1 ;
b) mesure de l'intensité de la coloration de l'échantillon après réaction enzymatique ;
c) comparaison de l'intensité de coloration mesurée à l'étape b) avec l'intensité mesurée pour des solutions standards ayant une teneur en glucose connue ;
d) détermination de la concentration en glucose dudit échantillon.

**14.** Procédé de dosage du glucose d'un échantillon, **caractérisé en ce qu'**il comprend les étapes suivantes :

a) introduction dans ledit échantillon d'une électrode à glucose selon la revendication 10;
b) mesure de l'intensité du courant dans l'échantillon ;
c) comparaison de l'intensité du courant mesurée à l'étape b) avec l'intensité mesurée pour des solutions standards ayant une teneur en glucose connue ;
d) détermination de la concentration en glucose dudit échantillon.

## Patentansprüche

**1.** Mutante der PQQ s-GDH, **dadurch gekennzeichnet, dass** die Aminosäure in Position 428 in Bezug auf die SEQ ID. Nr. 2 substituiert ist und die eine Aminosäuresequenz aufweist ausgewählt aus der Gruppe bestehend aus den SEQ. ID. NRn.: 4, 6, 8, 12 und 14.

**2.** Ein Nukleinsäuremolekül, **dadurch gekennzeichnet, dass** es eine Mutante der PQQ s-GDH gemäß Anspruch 1 kodiert.

**3.** Das Nukleinsäuremolekül gemäß Anspruch 2, **dadurch gekennzeichnet, dass** es durch Mutation des Nukleinsäuremolekül mit der Sequenz SEQ ID. Nr.: 1 erhalten wurde, mit Hilfe eines Paars von Oligonukleotiden ausgewählt aus der Gruppe bestehend aus den Paaren mit den SEQ. ID. Nrn.: 15 und 16; 19 und 20; 21 und 22; 23 und 24 sowie 25 und 26.

4. Das Nukleinsäuremolekül gemäß Anspruch 3, **dadurch gekennzeichnet, dass** es eine Sequenz aufweist ausgewählt aus der Gruppe bestehend aus SEQ. ID. Nrn.: 3, 5, 7, 11 und 13.

5. Ein Expressionsvektor, **dadurch gekennzeichnet, dass** er ein Nukleinsäuremolekül gemäß irgendeinem der Ansprüche 2 bis 4 enthält.

6. Eine Wirtszelle, die eine Mutante der PQQ s-GDH gemäß Anspruch 1 exprimiert, **dadurch gekennzeichnet, dass** sie mit einem Expressionsvektor gemäß Anspruch 5 transformiert ist.

7. Verwendung einer Mutante der PQQ s-GDH gemäß Anspruch 1 zur Messung der Glukosekonzentration in einer Probe.

8. Verwendung gemäß Anspruch 7, **dadurch gekennzeichnet, dass** die Probe eine biologische Probe, insbesondere Blut ist.

9. Glukose-Analyse-Kit, **dadurch gekennzeichnet, dass** es eine Mutante der PQQ s-GDH gemäß Anspruch 1 umfasst.

10. Eine Elektrode für Glukose, **dadurch gekennzeichnet, dass** sie ein leitendes Material aufweist, das mit einer Beschichtung überzogen ist, die wenigstens eine Mutante der PQQ s-GDH gemäß Anspruch 1 enthält.

11. Ein Glukosesensor, **dadurch gekennzeichnet, dass** er aus einer Elektrode gemäß Anspruch 10 besteht.

12. Eine Glukose-Biobatterie, **dadurch gekennzeichnet, dass** sie eine erste Elektrode gemäß Anspruch 10 als Anode und eine zweite Elektrode als Kathode aufweist.

13. Verfahren zur Bestimmung von in einer Probe gelösten Glukose, **dadurch gekennzeichnet, dass** es die folgenden Schritte umfasst:

    a) Einführen eines Oxidoreduktions-Reagenz, wobei die Reduktion zu einer Farbänderung führt, und einer Mutante der PQQ s-GDH gemäß Anspruch 1 in die Probe;
    b) Messen der Intensität der Färbung der Probe nach der enzymatischen Reaktion;
    c) Vergleich der in Schritt b) gemessenen Farbintensität mit der für Standardlösungen mit bekanntem Glukosegehalt gemessenen Intensität;
    d) Bestimmung der Glukosekonzentration in der Probe.

14. Ein Verfahren zur Bestimmung von Glukose in einer Probe, **dadurch gekennzeichnet, dass** es die folgenden Schritte umfasst:

    a) Einführen einer Glukoseelektrode gemäß Anspruch 10 in die Probe;
    b) Messung der Stromstärke in der Probe;
    c) Vergleichen der in Schritt b) gemessenen Stromstärke mit der für Standardlösungen mit bekanntem Glukosegehalt gemessenen Intensität;
    d) Bestimmung der Glukosekonzentration in der Probe.

## Claims

1. A mutant of PQQ s-GDH, **characterised in that** its amino acid located at position 428, referring to SEQ. ID. No. 2, is substituted, and it has an amino acid sequence selected from the group consisting of SEQ. ID. No. 4, 6, 8, 12 and 14.

2. A nucleic acid molecule, **characterised in that** it codes for a mutant PQQ s-GDH according to claim 1.

3. A nucleic acid molecule according to claim 2, **characterised in that** it is obtained by mutation of the nucleic acid molecule of sequence SEQ. ID. No. 1 with a pair of oligonucleotides selected from the group consisting of the pairs of SEQ. ID. No. 15 and 16; 19 and 20; 21 and 22; 23 and 24 and 25 and 26.

4. A nucleic acid molecule according to claim 3, **characterised in that** it has a sequence selected from the group consisting of SEQ. ID. No. 3, 5, 7, 11 and 13.

5. An expression vector, **characterised in that** it comprises a nucleic acid molecule according to any one of claims 2 to 4.

6. A host cell expressing a mutant of PQQ s-GDH according to claim 1, **characterised in that** it is transformed with an expression vector according to claim 5.

7. Use of a mutant of PQQ s-GDH according to claim 1 for measuring the concentration of glucose in a sample.

8. Use according to claim 7, **characterised in that** the sample is biological, in particular it is of blood.

9. A glucose assay kit, **characterised in that** it comprises a mutant of PQQ s-GDH according to claim 1.

10. A glucose electrode, **characterised in that** it comprises a conductive material covered with a deposit comprising at least one mutant of PQQ s-GDH according to claim 1.

11. A glucose sensor, **characterised in that** it consists of an electrode according to claim 10.

12. A glucose biofuel cell, **characterised in that** it comprises a first electrode according to claim 10 as an anode and a second electrode as a cathode.

13. A method of assaying glucose in solution in a sample, **characterised in that** it comprises the following steps:

a) introduction into said sample of a redox reagent, the reduction of which leads to a change in colour, and a mutant of PQQ s-GDH according to claim 1;
b) measurement of the intensity of the colouration of the sample after enzymatic reaction;
c) comparison of the intensity of colouration measured in step b) with the intensity measured for standard solutions having a known glucose content;
d) determination of the glucose concentration of said sample.

14. A method of assaying glucose in a sample, **characterised in that** it comprises the following steps:

a) introduction into said sample of a glucose electrode according to claim 10;
b) measurement of the intensity of the current in the sample;
c) comparison of the intensity of the current measured in step b) with the intensity measured for standard solutions having a known glucose content;
d) determination of the glucose concentration of said sample.

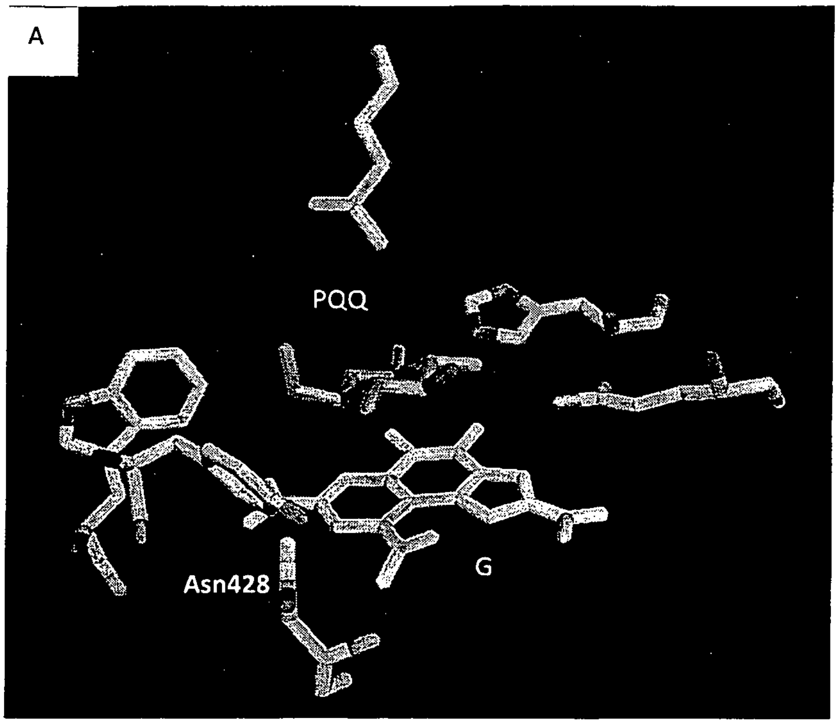


Figure 1A

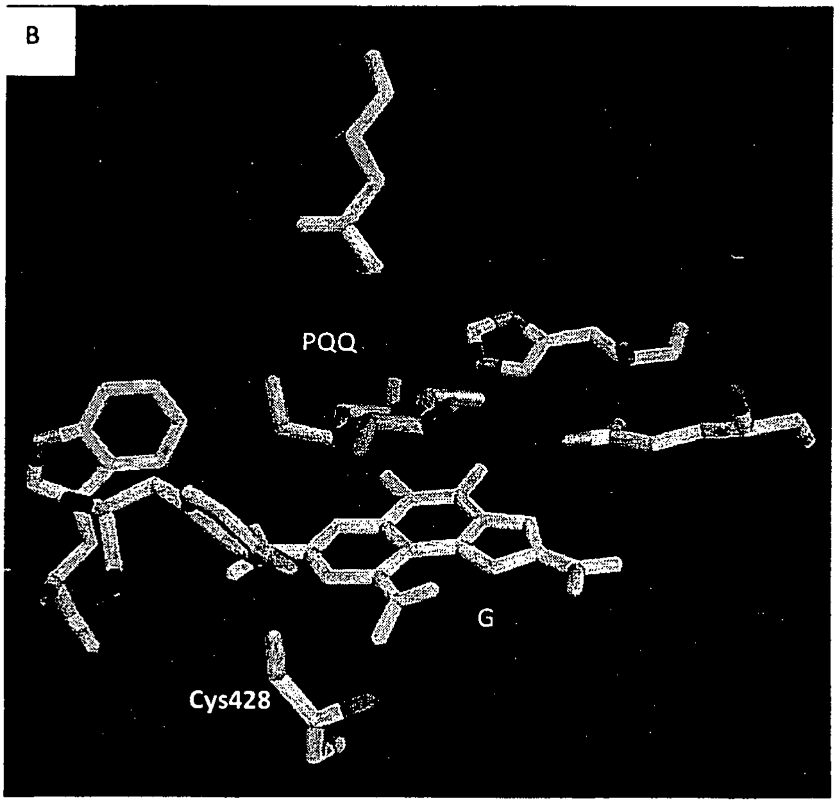


Figure 1B

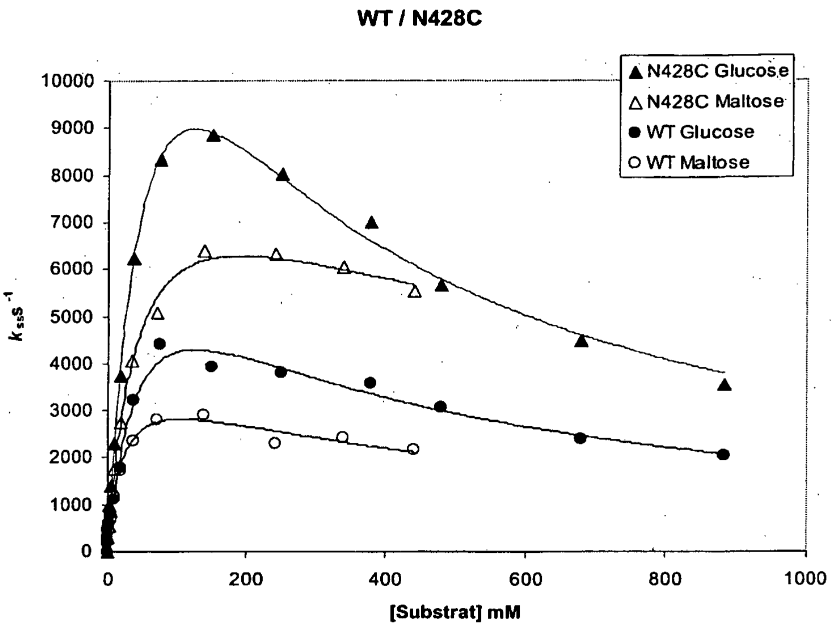


**Figure 2A**

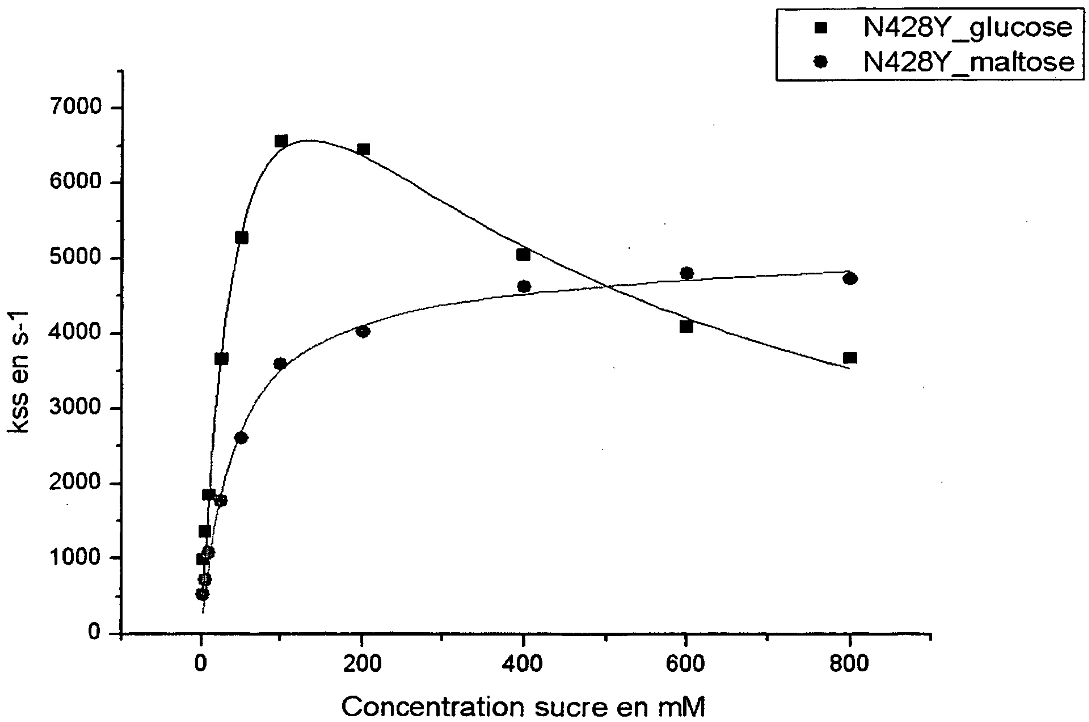


**Figure 2B**

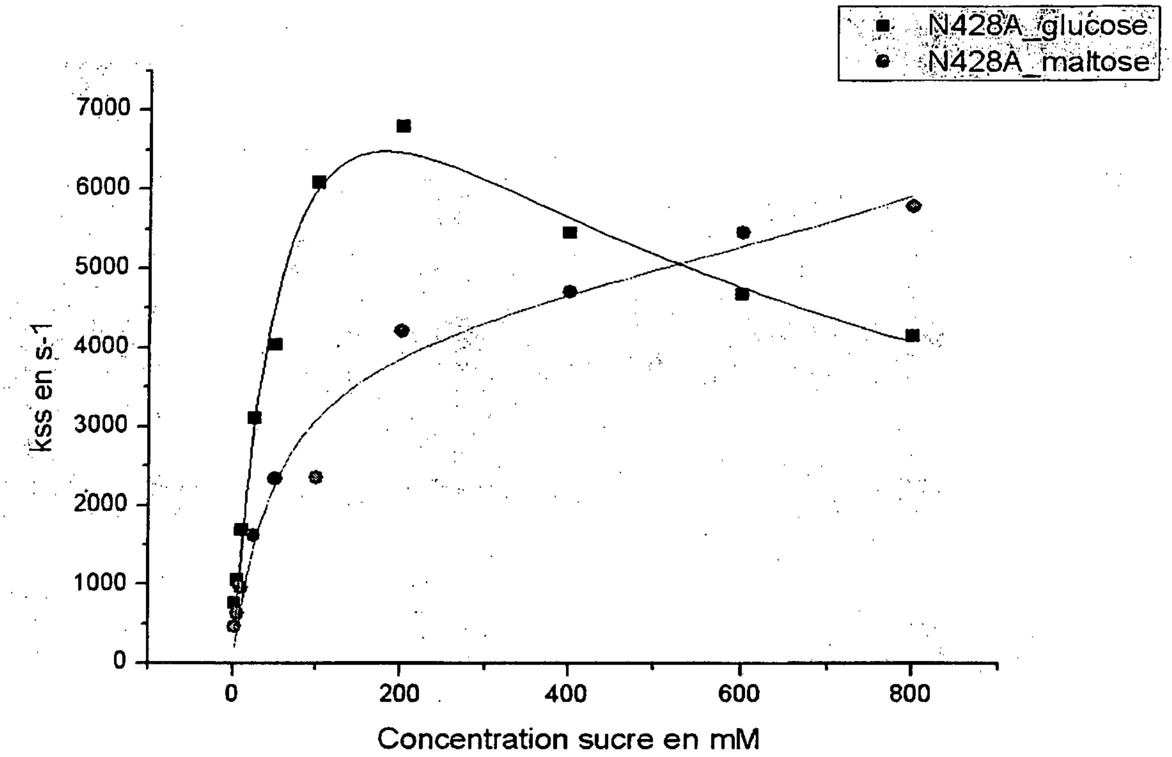


**Figure 2C**

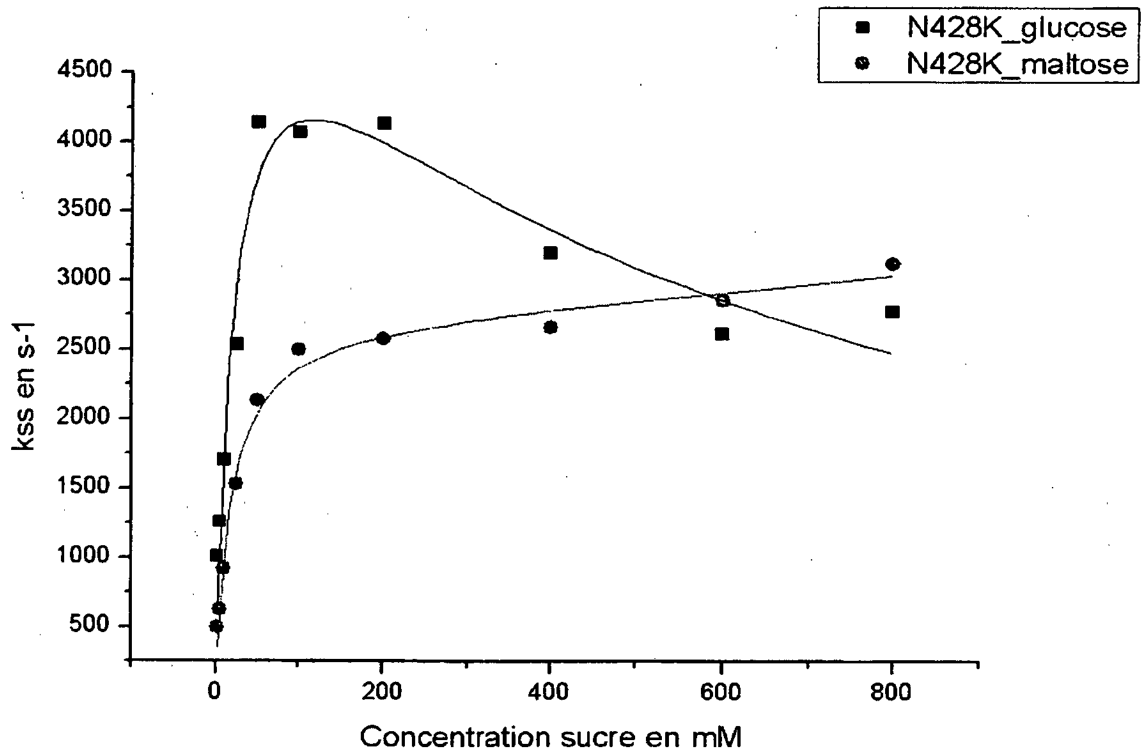


**Figure 2D**

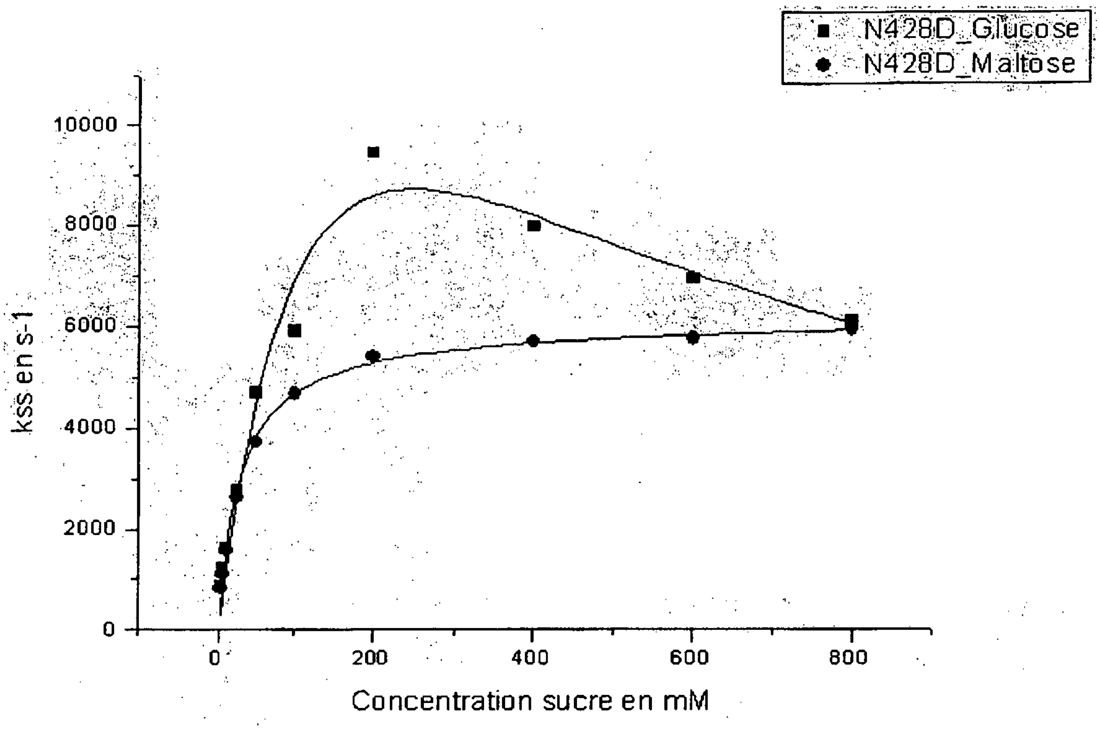


**Figure 2E**

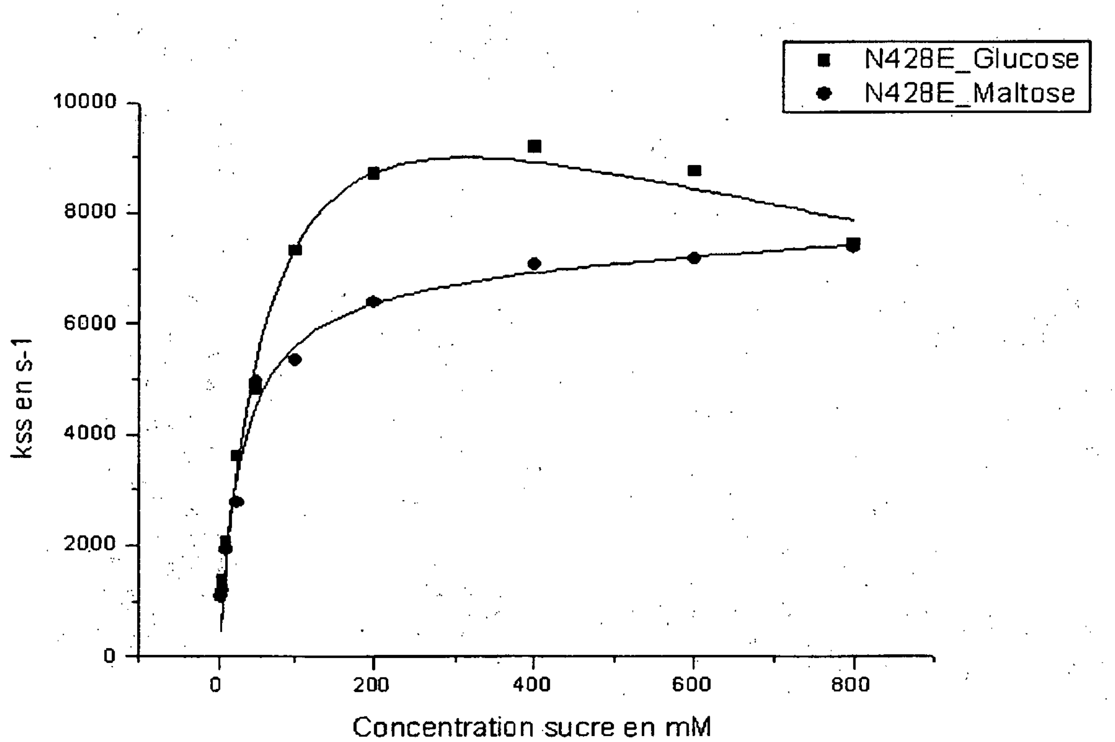


**Figure 2F**

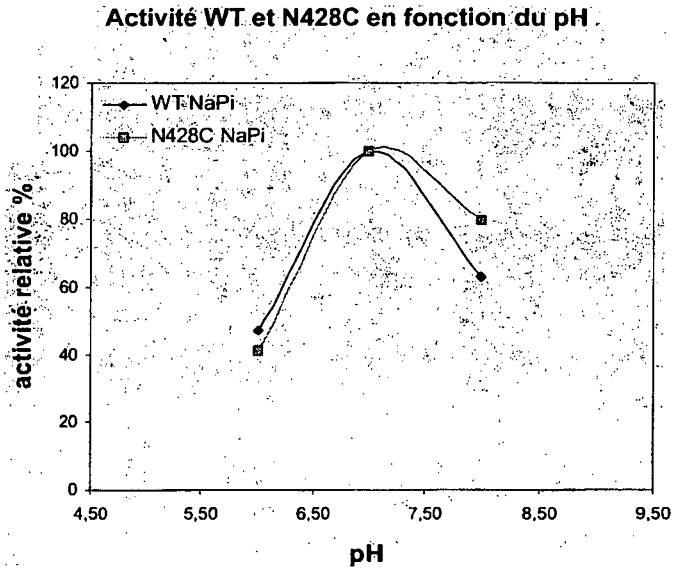


Figure 3A

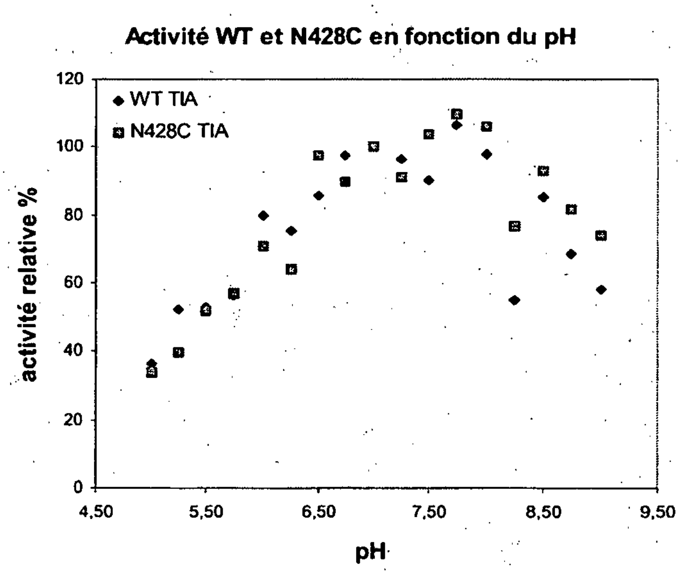


Figure 3B

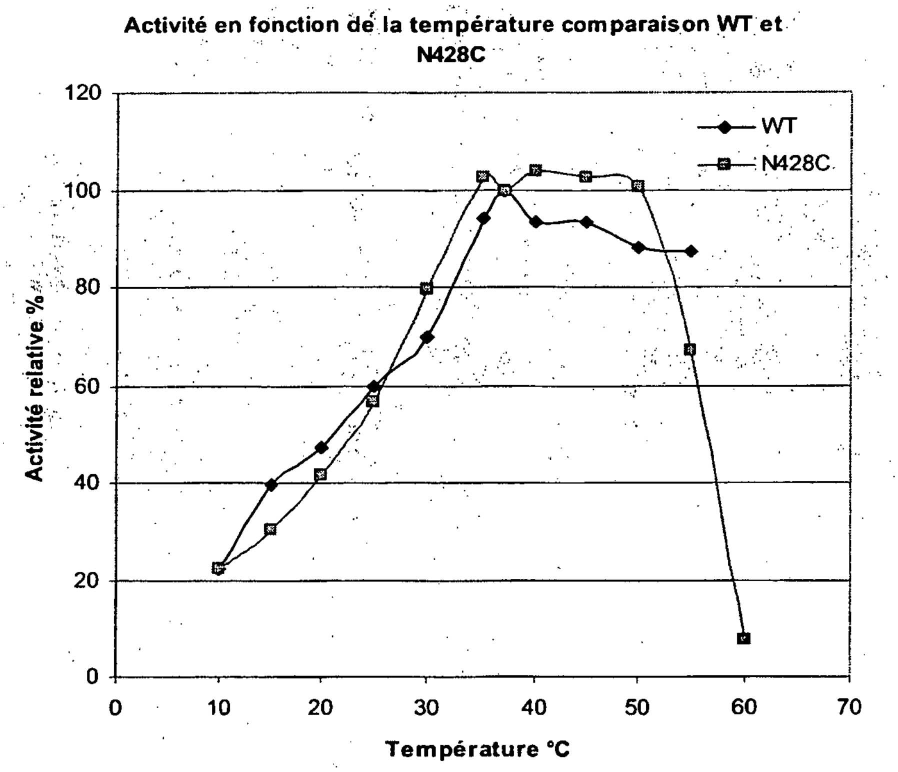


Figure 4

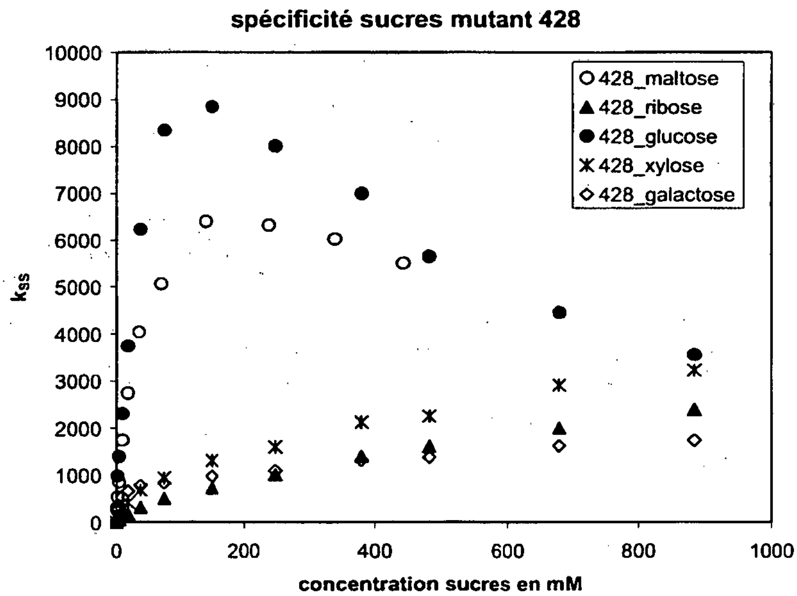


Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 7244600 B
- US 7244581 B
- US 20070105173 A
- EP 1367120 A
- US 7037698 B
- US 20070243566 A
- WO 2006085509 A
- EP 1666586 A
- US 7132270 B


### Littérature non-brevet citée dans la description


- **OUBRIÉ et al.** *J. Mol. Biol,* 1999, vol. 289, 319-333
- **IGARASHI et al.** *Archives of Biochemistry and Biophysocs,* 2004, vol. 428, 52-63
- **CLETON-JANSEN et al.** *Mol. Gen. Genet,* 1989, vol. 217, 430-436
- **SAMBROOK ; RUSSELL.** Molecular Cloning: A Laboratory Manual. Cold Spring Harbor Laboratory Press, 2001